# EUROPEAN PATENT APPLICATION

(11) **EP 4 023 648 A1**
(43) Date of publication of application: **06.07.2022**
(21) Application number: 20856096.1
(22) Date of filing: 27.08.2020
(51) Int. Cl.: C07D 487/04, C07D 261/02, A61K 31/519, A61P 35/00

(54) **CRYSTAL OF CARCINOGENIC FUSED KINASE INHIBITOR AND APPLICATIONS THEREOF**

(30) Priority: 28.08.2019 CN 201910804019; 18.09.2019 CN 201910883622
(71) Applicant: Simcere Pharmaceutical Co. Ltd., Jiangbei New District, Nanjing Jiangsu 210032 (CN)
(72) Inventor: WANG, Jianfei, Shanghai 200131 (CN); SUN, Jikui, Shanghai 200131 (CN); YANG, Guangwen, Shanghai 200131 (CN); ZHANG, Yang, Shanghai 200131 (CN)
(74) Representative: Krauss, Jan
(86) International application number: PCT/CN2020/111795
(87) International publication number: WO 2021/037156

(57) **Abstract**

Disclosed are a crystal of a carcinogenic fused kinase inhibitor and a preparation method therefor; also disclosed are applications of the crystal in preparing a medicament for treating tumor-related diseases.

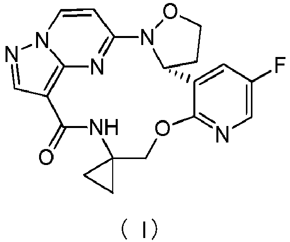

## Description

### The present application claims the following priorities:

CN201910804019.4, filed on August 28, 2019; and
CN201910883622.6, filed on September 18, 2019.

### TECHNICAL FIELD

The present invention relates to a crystal form of a carcinogenic fusion kinase inhibitor and a preparation method thereof, and applications of the crystal form in preparing a medicament for treating solid tumor-related diseases.

### BACKGROUND

Protein kinases are closely related to cell proliferation, differentiation, metabolism, apoptosis and the like. The oncogenic forms of protein kinases are abundantly expressed in a variety of different human tumor types and are highly responsive to some specific kinase inhibitors. Among them, anaplastic lymphoma kinase (ALK) is a receptor tyrosine kinase (RTK) belonging to the insulin receptor superfamily, is mainly expressed in the central and peripheral nervous system and plays a role in the normal development and function of the nervous system, and has been extensively studied in a large number of preclinical and clinical studies. ALK was first discovered in anaplastic large cell lymphoma (ALCL) as a continuously activated oncogenic form due to chromosomal translocation, which is a fusion protein NPM-ALK formed by fusion of the N-terminus of the normally expressed nucleolar phosphate protein NPM with the ALK kinase domain. At present, a variety of ALK fusion proteins have been identified, and are considered as potent oncogenic drivers of some tumors, such as inflammatory myofibroblastoma. Therefore, ALK fusion proteins also become important targets for cancer therapeutic interventions. Currently, several ALK inhibitors have entered into clinical trials and have been approved for marketing. Among them, Crizotinib was approved in 2011 for the treatment of patients with ALK positive non-small cell lung cancer (NSCLC). In 2014, Ceritinib was approved for the treatment of patients with ALK positive metastatic NSCLC. Although ALK inhibitors have been shown to be effective in the initial clinical setting, relapses have always been observed in treated patients, and ALK acquired resistance mutations occur. Among them, the emergence of brain metastatic tumors is an apparent reason for disease recurrence in patients treated with Crizotinib.

Tropomyosin-related kinases (Trk) are a class of nerve growth factor (NGF) receptors, which are highly expressed in neural cells. The Trk family consists of highly homologous tropomyosin-related kinase A (TrkA), tropomyosin-related kinase B (TrkB), and tropomyosin-related kinase C (TrkC), which are encoded by NTRK1, NTRK2, and NTRK3, respectively, and involve four ligands including NGF, BDNF, NT-4 and NT-3. By regulating the major signaling pathways such as PI3K-AKT, RAS-RAF-ERK, and PLCy-PKC, the Trk family is widely involved in important physiological activities such as cell proliferation, differentiation, survival, and neuronal growth. The continuously activated oncogenic form of Trk was first discovered from colorectal cancer as an oncogenic fusion gene (TPM3-NTRK1). Oncogenic Trk gene fusion does not require ligand activation to promote cancer cell proliferation and affect cancer-related downstream signaling pathways such as ERK and AKT. Drugs targeting TRK gene fusion, such as Entrectinib (RXDX-101) and Larotrectinib (LOXO-101), have also been shown to be effective in the initial clinical setting. However, acquired resistance mutations are also developed in treated patients under sustained action. New drugs targeting TRK gene fusion, such as TPX-0005 and LOXO-195 have partially addressed the problem of drug-resistant mutations.

Ros1 kinases are a class of receptor tyrosine kinases that have important effects on normal physiological functions. The continuously activated oncogenic forms of Ros1 fusion proteins were also found in a variety of human cancers, including glioblastoma, non-small cell lung cancer, colorectal cancer, and the like. A variety of drugs targeting Ros1 fusion proteins, such as Crizotinib, have been shown to be clinically effective. However, acquired resistance mutations have also been found in patients after sustained administration.

Therefore, for the clinical treatment of some cancers, there is an urgent need for a class of compounds that have inhibitory effects against multiple oncogenic fusion kinases and mutations thereof.

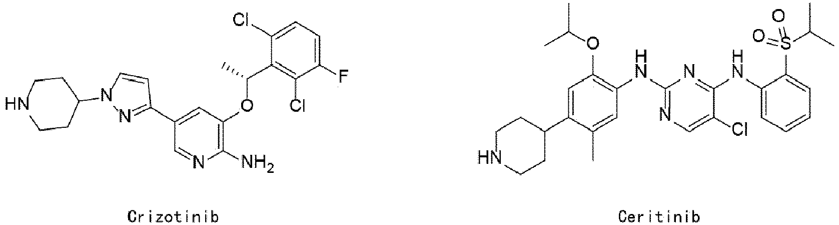

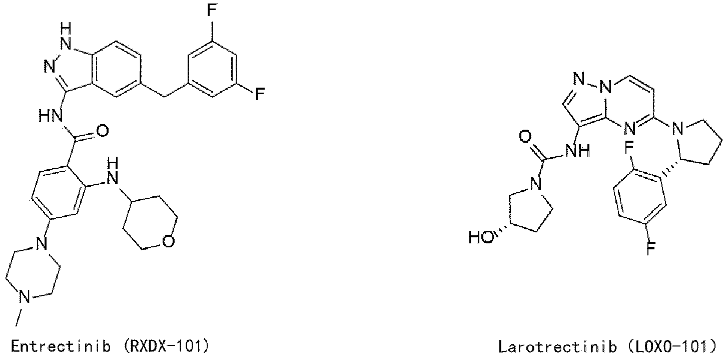

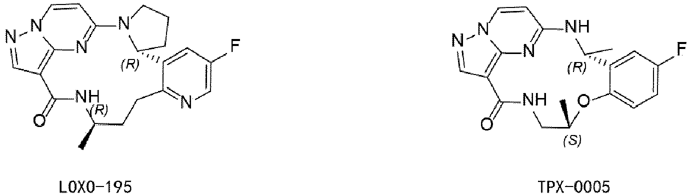

### CONTENTS OF THE INVENTION

The present invention provides the crystal form A of a compound of Formula (I), 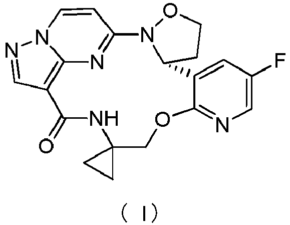 characterized in that an X-ray powder diffraction pattern of the crystal form A has characteristic diffraction peaks at the 2θ angels of 10.12°±0.20°, 19.03°±0.20°, and 19.74°±0.20°.

In some embodiments of the present invention, an X-ray powder diffraction pattern of the above crystal form A has characteristic diffraction peaks at the 2θ angels of 10.12±0.20°, 17.74±0.20°, 18.18±0.20°, 19.03±0.20°, 19.74±0.20°, 20.75±0.20°, 24.60±0.20°, and 28.21±0.20°.

In some embodiments of the present invention, an X-ray powder diffraction pattern of the above crystal form A has characteristic diffraction peaks at the 2θ angels of 10.115°, 17.740°, 18.178°, 19.033°, 19.738°, 20.749°, 21.609°, 22.411°, 23.345°, 24.160°, 24.597°, 25.787°, 28.211°, 30.613°, 31.133°, and 36.782°.

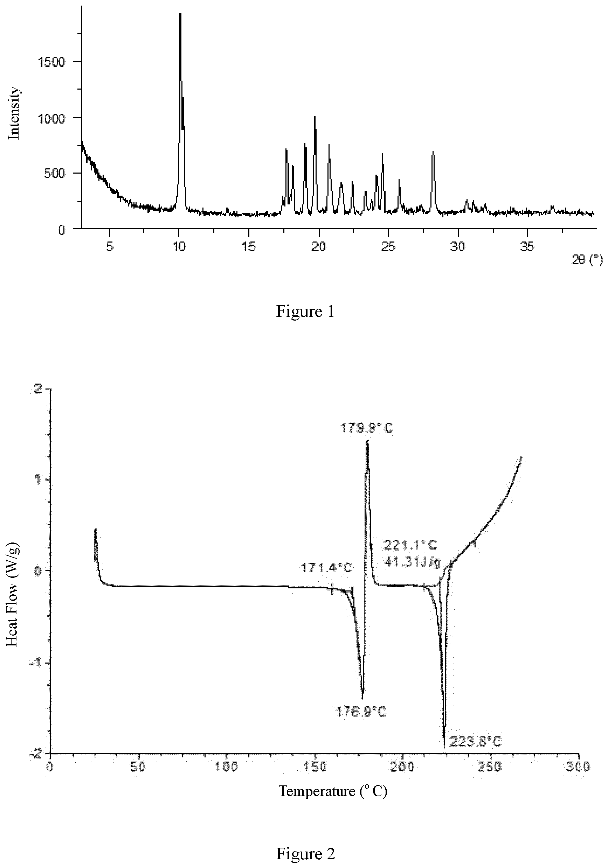

In some embodiments of the present invention, an XRPD pattern of the above crystal form A is shown as Figure 1.

In some embodiments of the present invention, the XRPD pattern analysis data of the above crystal form A are shown in Table 1.

**Table 1. XRPD Pattern Analysis Data of the Crystal Form A of the Compound of Formula (I)**

| No. | 2θ Angle(°) | Interplanar Distance (Å) | Relative Intensity (%) | No. | 2θ Angle(°) | Interplanar Distance (Å) | Relative Intensity (%) |
|---|---|---|---|---|---|---|---|
| 1 | 10.115 | 8.7448 | 100.0 | 9 | 23.345 | 3.8106 | 10.6 |
| 2 | 17.740 | 4.9998 | 33.1 | 10 | 24.160 | 3.6838 | 20.1 |
| 3 | 18.178 | 4.8803 | 25.5 | 11 | 24.597 | 3.6193 | 30.3 |
| 4 | 19.033 | 4.6629 | 36.5 | 12 | 25.787 | 3.4550 | 14.8 |
| 5 | 19.738 | 4.4979 | 49.5 | 13 | 28.211 | 3.1633 | 30.6 |
| 6 | 20.749 | 4.2811 | 35.1 | 14 | 30.613 | 2.9204 | 6.7 |
| 7 | 21.609 | 4.1127 | 14.9 | 15 | 31.133 | 2.8728 | 6.2 |
| 8 | 22.411 | 3.9673 | 16.6 | 16 | 36.782 | 2.4436 | 2.9 |

In some embodiments of the present invention, a differential scanning calorimetry curve of the above crystal form A has an onset of an endothermic peak at 171.4±3.0°C, and an onset of another endothermic peak at 221.1±3.0°C; and has a peak of an exothermic peak at 179.9±3.0°C.

In some embodiments of the present invention, a DSC pattern of the above crystal form A is shown as Figure 2.

In some embodiments of the present invention, a thermogravimetric analysis curve of the above crystal form A has a weight loss of 2.26% at 200.0°C±3.0°C.

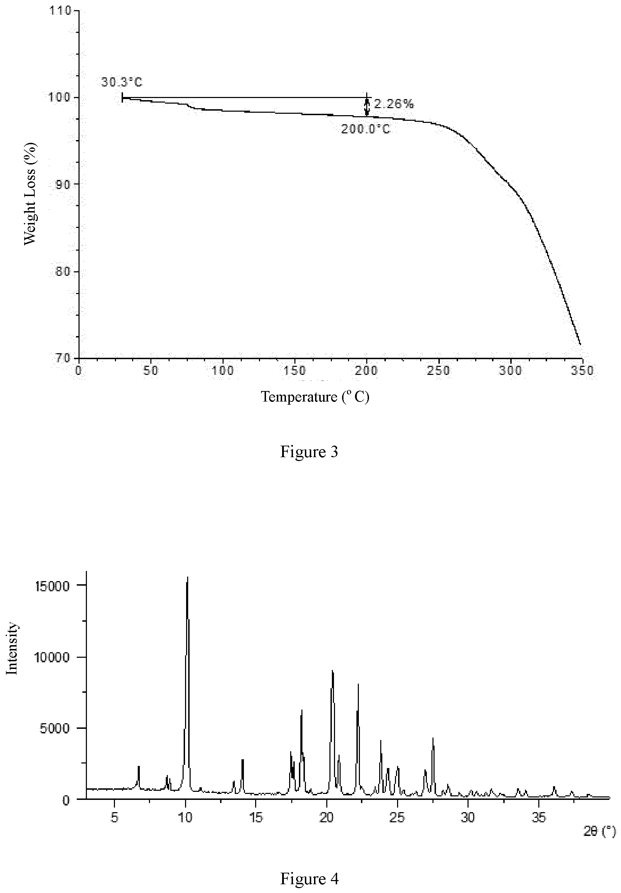

In some embodiments of the present invention, a TGA pattern of the above crystal form A is shown as Figure 3.

The present invention further provides a method for preparing the crystal form A of the compound of Formula (I), comprising:
1) adding pure water to the compound of Formula (I) to obtain a suspension; and
2) stirring the suspension at room temperature for 6 days and filtering the suspension.

The present invention further provides the crystal form B of a compound of Formula (I), 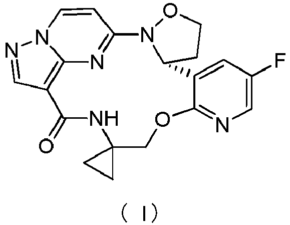 characterized in that an X-ray powder diffraction pattern of the crystal form B has characteristic diffraction peaks at the 2θ angels of 10.10±0.20°, 20.42±0.20°, and 22.21±0.20°.

In some embodiments of the present invention, an X-ray powder diffraction pattern of the above crystal form B has characteristic diffraction peaks at the 2θ angels of 10.10±0.20°, 14.04±0.20°, 17.48±0.20°, 18.20±0.20°, 20.42±0.20°, 22.21±0.20°, 23.83±0.20°, and 27.49±0.20°.

In some embodiments of the present invention, an X-ray powder diffraction pattern of the above crystal form B has characteristic diffraction peaks at the 2θ angels of 6.694°, 8.689°, 10.103°, 11.085°, 13.418°, 14.041°, 16.607°, 17.483°, 17.676°, 18.200°, 18.861°, 19.671°, 20.419°, 20.865°, 22.207°, 23.422°, 23.833°, 24.327°, 25.017°, 25.421°, 26.310°, 26.947°, 27.489°, 28.202°, 28.544°, 29.351°, 30.204°, 30.573°, 31.245°, 31.617°, 32.244°, 33.505°, 34.046°, 36.059°, 37.310°, and 38.534°.

In some embodiments of the present invention, an XRPD pattern of the above crystal form B is shown as Figure 4.

In some embodiments of the present invention, the XRPD pattern analysis data of the above crystal form B are shown in Table 2.

**Table 2. XRPD Pattern Analysis Data of the Crystal Form B of the Compound of Formula (I)**

| No. | 2θ Angle(°) | Interplanar Distance (Å) | Relative Intensity (%) | No. | 2θ Angle(°) | Interplanar Distance (Å) | Relative Intensity (%) |
|---|---|---|---|---|---|---|---|
| 1 | 6.694 | 13.2048 | 9.8 | 19 | 25.017 | 3.5595 | 14.1 |
| 2 | 8.689 | 10.1768 | 5.9 | 20 | 25.421 | 3.5038 | 2.9 |
| 3 | 10.103 | 8.7559 | 100.0 | 21 | 26.310 | 3.3874 | 2.5 |
| 4 | 11.085 | 7.9819 | 1.8 | 22 | 26.947 | 3.3088 | 12.4 |
| 5 | 13.418 | 6.5988 | 5.9 | 23 | 27.489 | 3.2448 | 27.4 |
| 6 | 14.041 | 6.3077 | 16.1 | 24 | 28.202 | 3.1644 | 2.7 |
| 7 | 16.607 | 5.3384 | 1.1 | 25 | 28.544 | 3.1272 | 5.6 |
| 8 | 17.483 | 5.0728 | 17.4 | 26 | 29.351 | 3.0430 | 1.9 |
| 9 | 17.676 | 5.0177 | 13.7 | 27 | 30.204 | 2.9590 | 2.9 |
| 10 | 18.200 | 4.8746 | 41.0 | 28 | 30.573 | 2.9242 | 2.7 |
| 11 | 18.861 | 4.7051 | 2.3 | 29 | 31.245 | 2.8628 | 2.0 |
| 12 | 19.671 | 4.5132 | 1.2 | 30 | 31.617 | 2.8299 | 3.6 |
| 13 | 20.419 | 4.3495 | 60.0 | 31 | 32.244 | 2.7763 | 1.8 |
| 14 | 20.865 | 4.2576 | 19.3 | 32 | 33.505 | 2.6746 | 4.0 |
| 15 | 22.207 | 4.0031 | 53.0 | 33 | 34.046 | 2.6334 | 3.2 |
| 16 | 23.422 | 3.7982 | 4.0 | 34 | 36.059 | 2.4909 | 5.2 |
| 17 | 23.833 | 3.7336 | 26.9 | 35 | 37.310 | 2.4102 | 2.6 |
| 18 | 24.327 | 3.6590 | 13.3 | 36 | 38.534 | 2.3364 | 1.2 |

In some embodiments of the present invention, a differential scanning calorimetry curve of the above crystal form B has an onset of an endothermic peak at 220.9±3.0°C.

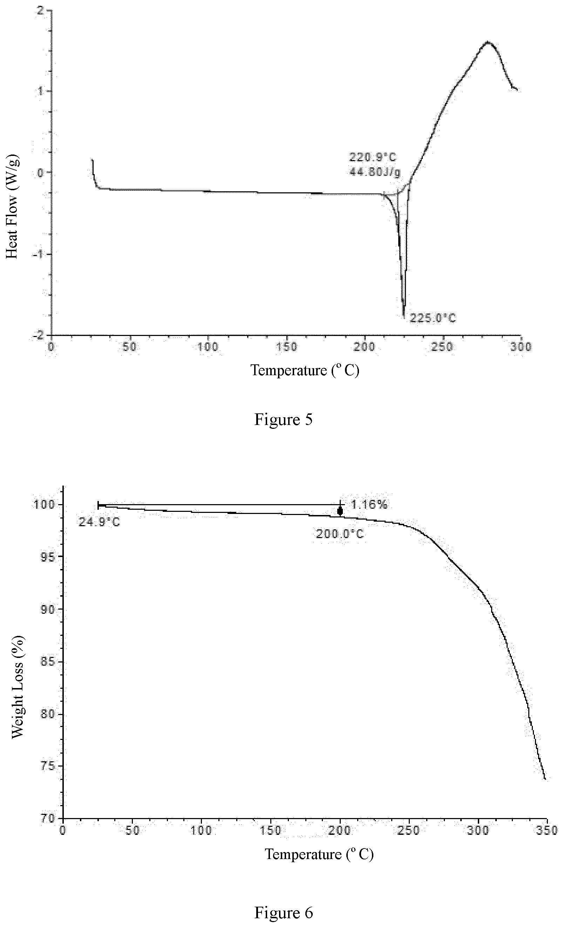

In some embodiments of the present invention, a DSC pattern of the above crystal form B is shown as Figure 5.

In some embodiments of the present invention, a thermogravimetric analysis curve of the above crystal form B has a weight loss of 1.16% at 200.0°C±3.0°C.

In some embodiments of the present invention, a TGA pattern of the above crystal form B is shown as Figure 6.

The present invention further provides the crystal form C of a compound of Formula (I), 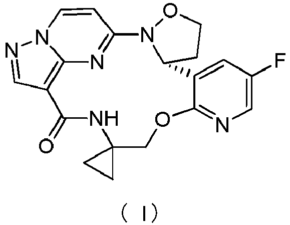 characterized in that an X-ray powder diffraction pattern of the crystal form C has characteristic diffraction peaks at the 2θ angels of 8.89°±0.20°, 13.37°±0.20°, and 20.98°±0.20°.

In some embodiments of the present invention, an X-ray powder diffraction pattern of the crystal form C has characteristic diffraction peaks at the 2θ angels of 8.89±0.20°, 12.86±0.20°, 13.37±0.20°, 14.71±0.20°, 18.58±0.20°, 20.98±0.20°, 21.85±0.20°, and 26.85±0.20°.

In some embodiments of the present invention, an X-ray powder diffraction pattern of the above crystal form C has characteristic diffraction peaks at the 2θ angels of 8.889°, 12.862°, 13.373°, 14.706°, 17.376°, 17.950°, 18.584°, 20.146°, 20.640°, 20.977°, 21.497°, 21.847°, 22.912°, 25.959°, and 26.853°.

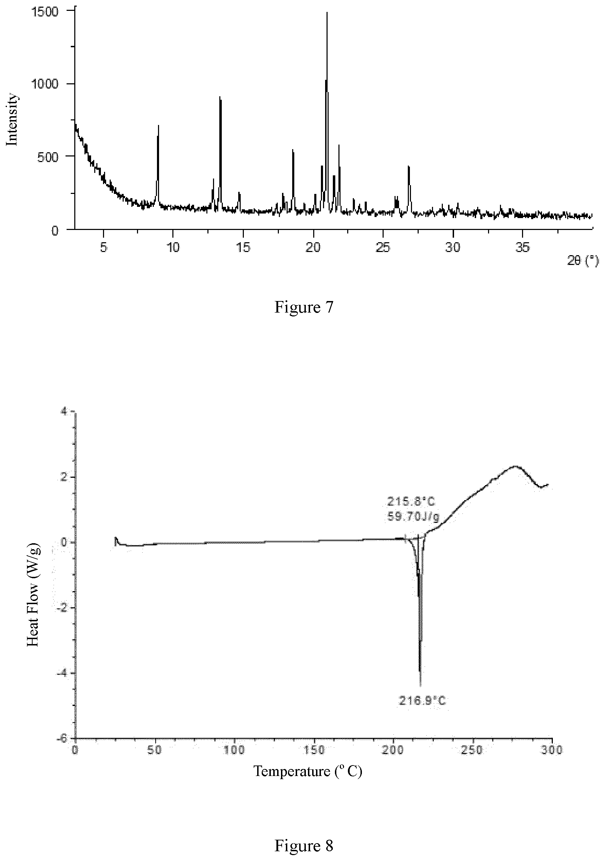

In some embodiments of the present invention, an XRPD pattern of the above crystal form C is shown as Figure 7.

In some embodiments of the present invention, the XRPD pattern analysis data of the above crystal form C are shown in Table 3.

**Table 3. XRPD Pattern Analysis Data of the Crystal Form C of the Compound of Formula (I)**

| No. | 2θ Angle(°) | Interplanar Distance (Å) | Relative Intensity (%) | No. | 2θ Angle(°) | Interplanar Distance (Å) | Relative Intensity (%) |
|---|---|---|---|---|---|---|---|
| 1 | 8.889 | 9.9479 | 32.6 | 9 | 20.640 | 4.3035 | 22.6 |
| 2 | 12.862 | 6.8831 | 15.3 | 10 | 20.977 | 4.2350 | 100.0 |
| 3 | 13.373 | 6.6211 | 42.7 | 11 | 21.497 | 4.1338 | 18.1 |
| 4 | 14.706 | 6.0238 | 9.5 | 12 | 21.847 | 4.0683 | 27.4 |
| 5 | 17.376 | 5.1038 | 3.7 | 13 | 22.912 | 3.8816 | 5.8 |
| 6 | 17.950 | 4.9419 | 4.8 | 14 | 25.959 | 3.4324 | 4.6 |
| 7 | 18.584 | 4.7745 | 32.2 | 15 | 26.853 | 3.3202 | 19.4 |
| 8 | 20.146 | 4.4078 | 9.5 | | | | |

In some embodiments of the present invention, a differential scanning calorimetry curve of the above crystal form C has an onset of an endothermic peak at 215.8±3.0°C.

In some embodiments of the present invention, a DSC pattern of the above crystal form C is shown as Figure 8.

In some embodiments of the present invention, a thermogravimetric analysis curve of the above crystal form C has a weight loss of 0.94% at 200.0°C±3.0°C.

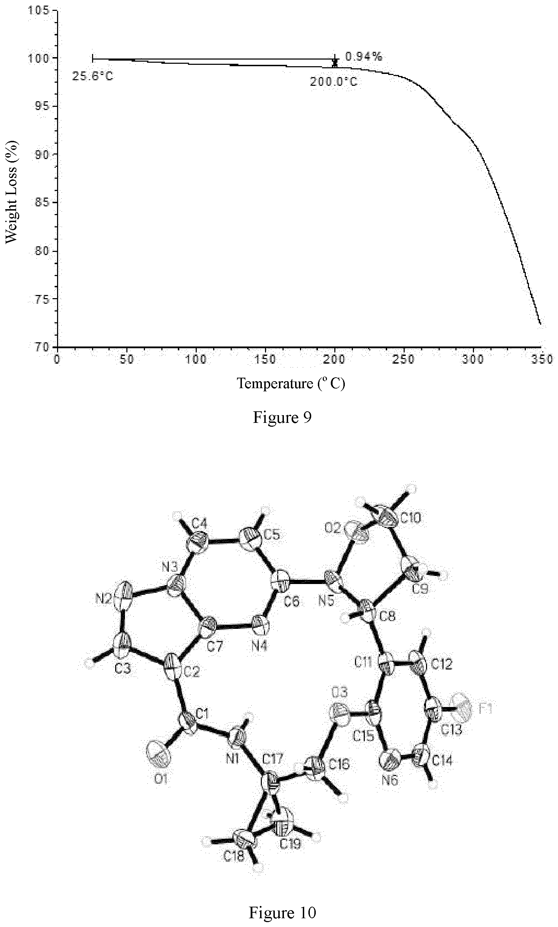

In some embodiments of the present invention, a TGA pattern of the above crystal form C is shown as Figure 9.

The present invention further provides a method for preparing the crystal form C of the compound of Formula (I), comprising a gas-solid permeation method, a suspension stirring method and a slow cooling method.

In some embodiments of the present invention, the above gas-solid permeation method comprises:
1) placing the compound of Formula (I) in a glass vial; and
2) placing the glass vial in an atmosphere of a solvent and then sealing the glass vial, allowing the solvent and the solid to induce crystallization,
wherein the solvent is methanol, ethyl acetate or acetone.

In some embodiments of the present invention, the suspension stirring method comprises:
1) formulating the compound of Formula (I) into a suspension in a solvent; and
2) slurrying the suspension at room temperature or 50°C,
wherein the solvent is methanol/water (v/v, 1:3), 1,4-dioxane/n-heptane (v/v, 1:4), dichloromethane/methyl tert-butyl ether (v/v, 1:9), anisole, isopropanol, isopropanol/water (v/v, 98:2), isopropanol/water (v/v, 94:6), isopropanol/water (v/v, 85:15), isopropyl acetate, ethanol/water (v/v, 1:3), methyl isobutyl ketone/n-heptane (v/v, 1:4), ethyl acetate/n-pentanol (v/v, 1:3), acetonitrile/methyl tert-butyl ether (v/v, 1:5), tetrahydrofuran/pure water (v/v, 1:9), chloroform/n-heptane (v/v, 1:5), or methyl ethyl ketone/methyl tert-butyl ether (v/v, 1:2); and the slurrying time is 2 hours to 144 hours.

In some embodiments of the present invention, the slow cooling method comprises:
1) formulating the compound of Formula (I) into a suspension in a solvent at 50°C;
2) stirring the suspension for 2 hours, filtering the suspension, and collecting a clear filtrate; and
3) cooling the clear filtrate from 50 °C to 5 °C at 0.1 °C/min,
wherein the solvent is isopropyl acetate.

The present invention further provides use of the crystal form A or the crystal form B or the crystal form C or the crystal forms obtained by the above methods in the preparation of a medicament for treating solid tumors.

### Technical Effects

Each of the crystal forms of the compound of the present invention is stable, is less affected by light, heat and humidity, has good efficacy for *in vivo* administration, and has a promising prospect for preparation into drugs. The compound of Formula (I) of the present invention exhibits a higher kinase inhibitory activity in a variety of kinases and mutants thereof, and shows a strong inhibition on mutations in the gate-keeping group region (gatekeeper), the solvent front region (solvent front mutation) and the DFG region of a variety of kinases. The compound of Formula (I) of the present invention has better pharmacokinetic properties in mice. After oral administration at the same dose, the total systemic exposure, peak concentration and bioavailability of the compound of the present invention are significantly superior to Larotrectinib (LOXO-101) and LOXO-195, showing excellent pharmacokinetic properties. The compound of Formula (I) of the present invention exhibits significant anti-tumor effects.

### Definitions and Description

Unless otherwise indicated, the following terms and phrases as used herein are intended to have the following meanings. A particular term or phrase without a particular definition should not be regarded as being indefinite or unclear, but should be understood in its ordinary sense. When a tradename is used herein, it is intended to refer to the corresponding commodity or its active ingredient.

The intermediate compounds of the present invention can be prepared through many synthetic methods which are well-known to the person skilled in the art, including the following illustrative specific embodiments, embodiments obtained by combining the illustrative specific embodiments with other chemical synthetic methods and the equivalent alternative methods which are well-known to the person skilled in the art. The preferred embodiments include but not limited to the examples of the present invention.

The chemical reactions in the specific embodiments of the present invention are carried out in suitable solvents which should be adapted to the chemical changes of the present invention and desired reagents and materials thereof. In order to obtain the compound of the present invention, it is sometimes necessary for the person skilled in the art to modify or select synthetic steps or reaction schemes based on the existing embodiments.

The structure of the compound of the present invention can be confirmed by conventional methods well known to the person skilled in the art. If the present invention relates to the absolute configuration of a compound, the absolute configuration can be confirmed by conventional technical means in the art. For example, in the single crystal X-ray diffraction (SXRD) method, the absolute configuration can be confirmed by collecting diffraction intensity data of a cultured single crystal using Bruker D8 venture diffractometer, in which the light source is CuKα radiation and the scanning mode is ϕ/ω scanning, and further resolving the crystal structure by the direct method (Shelxs97) after collecting the relevant data.

Hereinafter, the present invention will be described in detail by means of examples, which are not intended to limit the present invention in any way.

All solvents used in the present invention are commercially available and can be used without further purification.

The solvents used in the present invention are commercially available. The following abbreviations are used in the present invention: EtOH represents ethanol; MeOH represents methanol; TFA represents trifluoroacetic acid; TsOH represents p-toluenesulfonic acid; mp represents melting point; EtSO₃H represents ethanesulfonic acid; MeSO₃H represents methanesulfonic acid; THF represents tetrahydrofuran; EtOAc represents ethyl acetate.

Compounds are named according to conventional nomenclature principles in the art or using ChemDraw^{®} software. Commercially available compounds are named using supplier catalog names.

### X-ray Powder Diffraction (X-ray powder diffractometer, XRPD) Method of the Present Invention

Instrument Model: PANalytical Empyrean type X-ray powder diffractometer
Test Method: Approximately 10 mg of sample is used for XRPD detection.
The detailed XRPD parameters are as follows:
   Ray Source: Cu, kα (Kα1=1.540598 Å, Kα2=1.544426 Å, Kα2/Kα1 intensity ratio: 0.5)
   Light Tube Voltage: 45 kV, Light Tube Current: 40 mA
   Divergence Slit: Automatic
   Scan Mode: Continuous
   Scan Angle Range: 3-40 deg
   Scan Time per Step: 17.780 seconds
   Step Size: 0.0167 degrees
   Instrument Model: PANalytical X'Pert³ type X-ray powder diffractometer
   Test Method: Approximately 10 mg of sample is used for XRPD detection.
   The detailed XRPD parameters are as follows:
      Ray Source: Cu, kα (Kα1=1.540598 Å, Kα2=1.544426 Å, Kα2/Kα1 intensity ratio: 0.5)
      Light Tube Voltage: 45 kV, Light Tube Current: 40 mA
      Divergence Slit: 1/16 degrees
      Scan Mode: Continuous
      Scan Angle Range: 3-40 deg
      Scan Time per Step: 46.665 seconds
      Step Size: 0.0263 degrees

### Differential Scanning Calorimetry (Differential Scanning Calorimeter, DSC) Analysis Method of the Present Invention

Instrument Model: TA Instruments Q200/Discovery DSC 2500 Differential Scanning Calorimeter

Test Method: A sample (1 to 10 mg) is taken and placed in a DSC aluminum pan for testing. The sample is heated from room temperature to 300°C at a heating rate of 10°C/min under the condition of 50 mL/min of N₂.

### Thermal Gravimetric Analysis (Thermal Gravimetric Analyzer, TGA) Method of the Present Invention

Instrument Model: TA Instruments Q5000/Discovery TGA 5500 Thermal Gravimetric Analyzer

Test Method: A sample (2 to 15 mg) is taken and placed in a TGA aluminum pan for testing. The sample is heated from room temperature to 350°C at a heating rate of 10°C/min under the condition of 50 mL/min of N₂.

### BRIEF DESCRIPTION OF THE DRAWINGS

Figure 1 is an XRPD pattern using Cu-Ka radiation of the crystal form A of the compound of Formula (I).
Figure 2 is a DSC pattern of the crystal form A of the compound of Formula (I).
Figure 3 is a TGA pattern of the crystal form A of the compound of Formula (I).
Figure 4 is an XRPD pattern using Cu-Ka radiation of the crystal form B of the compound of Formula (I).
Figure 5 is a DSC pattern of the crystal form B of the compound of Formula (I).
Figure 6 is a TGA pattern of the crystal form B of the compound of Formula (I).
Figure 7 is an XRPD pattern using Cu-Ka radiation of the crystal form C of the compound of Formula (I).
Figure 8 is a DSC pattern of the crystal form C of the compound of Formula (I).
Figure 9 is a TGA pattern of the crystal form C of the compound of Formula (I).
Figure 10 is an ellipsoid diagram of the stereostructure of the compound of Formula (I).
Figure 11 is a unit cell stacking diagram of the compound of Formula (I) along the a-axis direction.
Figure 12 is *in vivo* efficacy results of the compound of Formula (I) on human colon cancer cell line KM12 cells subcutaneous xenograft tumor in a BALB/c mouse model.
Figure 13 is *in vivo* efficacy results of the compound of Formula (I) on human lung cancer LU-01-0414 subcutaneous xenograft tumor in a BALB/c mouse model.

### EMBODIMENTS

In order to better understand the contents of the present invention, the present invention will be further described in conjunction with the specific examples, but the specific embodiments are not limitations to the contents of the present invention.

### Example 1: Preparation of Compound of Formula (I)

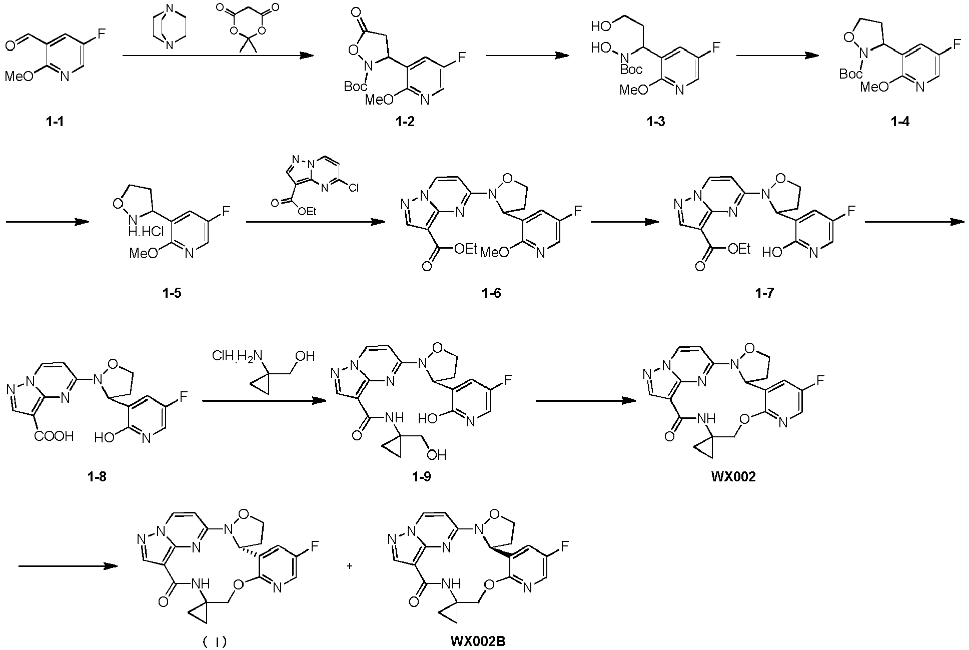

### Step 1: Synthesis of Compound 1-2

Compound 1-1 (15 g, 96.70 mmol, 1 *eq)* was dissolved in ethyl acetate (300 mL). Isopropylidene malonate (13.94 g, 96.70 mmol, 1 *eq),* triethylene diamine (1.08 g, 9.67 mmol, 1.06 mL, 0.1 *eq)* and tert-butyl N-hydroxycarbamate (12.87 g, 96.70 mmol, 1 *eq)* were added, and the resulting reaction solution was stirred at 25°C for 16 h. The reaction solution was washed twice with water (200 mL each time), and then washed once more with 100 mL saturated brine. The organic phase was dried over anhydrous sodium sulfate and filtered to remove the drying agent. The solvent was removed from the filtrate under a reduced pressure to give a crude product. The crude product was purified by column (petroleum ether: ethyl acetate =3:1) to give Compound 1-2. ¹H NMR (400MHz, CDCl₃) δ: 7.98 (d, J = 2.8 Hz, 1H), 7.52-7.48 (m, 1H), 5.65 (dd, J = 3.6, 10.0 Hz, 1H), 3.95 (s, 3H), 3.33 (dd, J = 9.6, 18.0 Hz, 1H), 2.74 (dd, J = 3.6, 16.0 Hz, 1H), 1.50 (s, 9H). LCMS m/z=313.3 [M+H] ⁺.

### Step 2: Synthesis of Compound 1-3

Compound 1-2 (21.40 g, 68.53 mmol, 1 *eq)* was dissolved in tetrahydrofuran (300 mL), lithium borohydride (4.48 g, 205.58 mmol, 3 *eq)* was added thereto slowly, and the resulting mixture was stirred at 25°C for 0.1 h. 200 mL water was added to the reaction solution and then extracted twice with ethyl acetate (50 mL each time). The combined organic phase was washed with 100 mL saturated brine, then dried over anhydrous sodium sulfate and filtered to remove the drying agent. The filtrate was spin-dried to give crude Compound 1-3. ¹H NMR (400 MHz, CDCl₃) δ: 7.88 (d, J = 3.2 Hz, 1 H), 7.67-7.64 (dd, J = 2.8, 8.8 Hz, 1 H), 5.46-5.42 (m, 1 H), 3.91 (s, 3 H), 3.86-3.71 (m, 2 H), 2.24-2.14 (m, 1 H), 2.08-2.00 (m, 1 H), 1.41 (s, 9 H). LCMS m/z=317.3 [M+H]⁺.

### Step 3: Synthesis of Compound 1-4

Compound 1-3 (14.52 g, 45.90 mmol, 1 *eq)* and triphenylphosphine (30.10 g, 114.76 mmol, *2.5 eq)* were dissolved in tetrahydrofuran (150 mL). The resulting reaction solution was cooled to 5°C with an ice-water bath, and then diisopropyl azodicarboxylate (27.85 g, 137.71 mmol, 26.77 mL, 3 *eq)* was added dropwise. After the dropwise addition was completed, the ice bath was removed and the reaction solution was stirred at 25°C for 0.1 h. The reaction solution was spin-dried and the residue was purified by column (petroleum ether: ethyl acetate =50:1~30:1~10:1~5:1) to give Compound 1-4. ¹H NMR (400 MHz, CDCl₃) δ: 7.88 (d, J = 3.2 Hz, 1 H), 7.52-7.50 (m, 1 H), 5.38-5.35 (m, 1H), 4.13-4.03 (m, 1H), 3.94 (s, 3H), 3.89-3.82 (m, 1H), 2.84-2.76 (m, 1H), 2.12-2.03 (m, 1H), 1.50 (s, 9H). LCMS m/z=299.3[M+H]⁺.

### Step 4: Synthesis of Compound 1-5

Compound 1-4 (3.00 g, 10.06 mmol, 1 *eq)* was dissolved in a solution of hydrogen chloride in methanol (4 M, 12.57 mL, 5 *eq)* and the resulting reaction solution was stirred at 25°C for 3 h. The reaction solution was spin-dried to give Compound 1-5. ¹H NMR (400 MHz, CD₃OD) δ: 8.17 (d, J = 2.8 Hz, 1 H), 7.81-7.79 (m, 1 H), 5.21 (t, J = 8.0 Hz, 1 H), 4.60-4.54 (m, 1 H), 4.40-4.32 (m, 1 H), 4.04 (s, 3 H), 2.96-2.80 (m, 2 H). LCMS m/z=199.3 [M+H] ⁺.

### Step 5: Synthesis of Compound 1-6

Ethyl 5-chloropyrazolo[1,5-a] pyrimidine-3-carboxylate (1.92 g, 8.52 mmol, 1 *eq),* Compound 1-5 (2.20 g, 9.38 mmol, 1.1 *eq)* and n-butanol (5 mL) were added successively into a long tube, and then N, N-diisopropylethylamine (6.61 g, 51.14 mmol, 8.91 mL, 6 *eq)* was added thereto. The resulting reaction solution was stirred at 90°C for 3.5 h. The reaction solution was concentrated, 30 mL water was added, and then the resulting mixture was extracted with 30 mL ethyl acetate. The organic phase was separated and washed once with 20 mL saturated brine, then dried over anhydrous sodium sulfate, and filtered to remove the drying agent. The solvent was removed from the filtrate under a reduced pressure to give a crude product. The crude product was purified by column (petroleum ether: ethyl acetate =100:0~10:1~5:1~2:3) to give Compound 1-6. ¹H NMR (400 MHz, CDCl₃) δ: 8.48 (d, J = 7.6 Hz, 1 H), 8.39 (s, 1 H), 7.92 (d, J = 3.2 Hz, 1 H), 7.58-7.55 (m, 1 H), 7.03 (d, J = 7.6 Hz, 1 H), 6.06 (dd, J = 5.2, 8.8 Hz, 1 H), 4.33-4.24 (m, 2 H), 4.22-4.18 (m, 1 H), 4.01 (s, 3 H), 3.93 - 3.87 (m, 1 H), 2.94-2.90 (m, 1 H), 2.36-2.30 (m, 1 H), 1.27 (t, J=6.8 Hz, 3 H). LCMS m/z=388.3 [M+H] ⁺

### Step 6: Synthesis of Compound 1-7

Compound 1-6 (3.9 g, 10.07 mmol, 1 *eq)* was dissolved in acetonitrile (100 mL), sodium iodide (4.53 g, 30.20 mmol, 3 *eq)* was added, and trimethylchlorosilane (3.28 g, 30.20 mmol, 3.83 mL, 3 *eq)* was added dropwise under stirring. After the addition was completed, the resulting reaction solution was stirred at reflux for 0.5 h at 75°C under nitrogen protection. 50 mL water was added to the reaction solution to precipitate a solid, which was filtered and the filter cake was dried in vacuum at 40°C to give Compound 1-7. ¹H NMR (400 MHz, CD₃OD) δ: 8.69 (d, J = 7.6 Hz, 1 H), 8.30 (s, 1 H), 7.63-7.60 (m, 1 H), 7.38 (t, J = 3.2 Hz, 1 H), 7.10 (d, J = 5.6, 1 H), 5.79-5.75 (m, 1 H), 4.26-4.19 (m, 2 H), 2.98-2.90 (m, 1 H), 2.35-2.29 (m, 1 H), 1.25 (t, J = 7.2 Hz, 3 H). LCMS m/z=374.3 [M+H]⁺.

### Step 7: Synthesis of Compound 1-8

Compound 1-7 was dissolved in methanol (30 mL), and a solution of sodium hydroxide (385.68 mg, 9.64 mmol, 4 *eq)* in water (3 mL) was added thereto. The resulting reaction solution was stirred for 16 h at 60°C under nitrogen protection. The reaction solution was cooled to room temperature, adjusted to a pH of about 7 with a 2M hydrochloric acid solution, and then directly spin-dried to give Compound 1-8, which was used directly in the next step. LCMS m/z=346.2 [M+ H]⁺.

### Step 8: Synthesis of Compound 1-9

Compound 1-8 was dissolved in N,N-dimethylformamide (8 mL), then N,N-diisopropylethylamine (449.36 mg, 3.48 mmol, 605.60 µL, 3.5 *eq)* and O-(7-azabenzotriazol-1-yl) -N, N, N, N-tetramethyluronium hexafluorophosphate (453.26 mg, 1.19 mmol, 1.2 *eq)* were added and stirred for 0.5 h, and then 1-(hydroxymethyl) cyclopropylamino hydrochloride (159.59 mg, 1.29 mmol, 1.3 *eq*, HCl) was added and the resulting reaction solution was reacted at 25°C for 3 h. The reaction solution was poured into 80 mL of a saturated aqueous ammonium chloride solution, and then extracted with dichloromethane (60 mL ^{∗}3), and the combined organic phase was washed with saturated brine (60 mL^{∗}3). The organic phase was dried over an appropriate amount of anhydrous sodium sulfate, and filtered to remove the drying agent. The filtrate was concentrated to give a crude product. 2 mL water was added to the crude product, followed by lyophilization to give Compound 1-9, which was used directly in the next reaction. LCMS m/z=415.3 [M+H]⁺.

### Step 9: Synthesis of Compound of Formula (I) and WX002B

Compound 1-9 (200 mg, 482.64 µmol, 1 *eq)* was dissolved in tetrahydrofuran (2 mL), and then tri-n-butylphosphine (195.29 mg, 965.28 µmol, 238.16 µL, 2 *eq)* was added. The resulting reaction solution was cooled to 0°C, then azodicarbonyldipiperidine (243.55 mg, 965.28 µmol, 2 *eq)* was added, and the resulting reaction solution was reacted at 25°C for 4 h. The reaction solution was directly drained to dryness. The residue was subsequently purified by flash silica gel column (petroleum ether/ethyl acetate =0~90%) and thin layer chromatography silica gel plate (ethyl acetate: methanol =10:1) to give Compound WX002. WX002 was resolved via SFC (Chromatography column: DAICEL CHIRALCEL OD-H (250mm^{∗}30mm, 5µm); Mobile phase: A (CO₂) and B (methanol with 0.1% aqueous ammonia; Gradient: B%=32%-32%, 7.5min), to give the compound of Formula (I) and WX002B.

The compound of Formula (I): ¹H NMR (400MHz, CDCl₃) δ: 9.27 (s, 1H), 8.42 (d, J = 7.6 Hz, 1H), 8.30 (s, 1H), 7.97 (d, J = 2.8 Hz, 1H), 7.59-7.57 (m, 1H), 6.79 (d, J = 8.0 Hz, 1H), 6.11 (t, J = 8.4 Hz, 1H), 4.88 (d, J = 10.8 Hz, 1H), 4.53 (t, J = 8.0 Hz, 1H), .33.97-3.90 (m, 1H), 3.84 (d, J = 10.8 Hz, 1H), 3.08-3.01 (m, 1H), 2.60-2.46 (m, 1H), 2.39-2.33 (m, 1H), 1.48-1.42 (m, 1H), 0.95-0.90 (m, 1H), 0.87-0.81 (m, 1H). LCMS m/z=397.3 [M+ H] ⁺.

SFC (Chromatography column: Chiralcel OD-3, 3 µm , 0.46 cm id × 10cm L; Mobile phase: A (CO₂) and B (MeOH with 0.05% isopropylamine); Gradient: B%=5~40%, 5 min; Flow rate: 4.0 mL/min; Wavelength: 220nm; Pressure: 100 bar, Rt=2.14 min, chiral isomer excess 100%).

WX002B: 1H NMR (400MHz, CDCl₃) δ: 9.27 (s, 1H), 8.41 (d, J = 7.6 Hz, 1H), 8.30 (s, 1H), 7.97 (d, J = 2.8 Hz, 1H), 7.59-7.56 (m, 1H), 6.79 (d, J = 7.6 Hz, 1H), 6.13-6.09 (m, , 1H), 4.88 (dd, J = 10.8, 1.6 Hz, 1H), 4.53 (t, J = 8.0 Hz, 1H), 3.97-3.90 (m, 1H), 3.84 (d, J = 10.8 Hz, 1H), 3.08-3.01 (m, 1H), 2.60-2.49 (m, 1H), 2.39-2.33 (m, 1H), 1.48-1.42 (m, 1H), 0.96-0.90 (m, 1H), 0.87-0.81 (m, 1H). LCMS m/z=397.3 [M+ H] +.

SFC (Chromatography column: Chiralcel OD-3, 3 µm, 0.46 cm id × 10cm L; Mobile phase: A (CO₂) and B (MeOH with 0.05% isopropylamine); Gradient: B%=5~40%, 5 min; Flow rate: 4.0 mL/min; Wavelength: 220nm; Pressure: 100 bar, Rt=2.49 min, chiral isomer excess 100%).

### Example 2: Preparation of Crystal Form A of Compound of Formula (I)

### Method 1: Anti-solvent addition method

Each about 15 mg of the compound of Formula (I) was weighed and placed into a 5 mL glass vial, and a corresponding volume of a positive solvent was added to give a clear solution. A corresponding anti-solvent was added dropwise thereto under magnetic stirring (rotational speed of about 1000 revolutions per minute), and if a solid was precipitated, the resulting solid was centrifuged and separated. If no solid was precipitated after addition of about 4 mL of the anti-solvent, the addition of the anti-solvent was stopped. The sample was transferred to an environment at -20°C for magnetic stirring or to a room temperature condition for volatilization in open until a solid was precipitated. The resulting solid was separated, dried and then tested for XRPD. The experimental results were as follows.

| Test No. | Solid Mass (mg) | Positive Solvent/ Volume (mL) | Anti-solvent/ Volume (mL) | Phenomenon After Dropwise Addition of Anti-solvent | Crystal Form |
|---|---|---|---|---|---|
| 1 | 14.8 | acetone/0.4 | n-heptane/3. 0 | a solid was precipitated immediately after the anti-solvent was added dropwise | Crystal Form A |
| 2 | 15.4 | 1,4-dioxane/0.4 | methyl tert-butyl ether/4.0 | no solid was precipitated after the anti-solvent was added dropwise, and a solid was precipitated after the solution was transferred to room temperature for volatilization in open for about three days. | Crystal Form A |
| 3 | 15.5 | acetonitrile/0.6 | isopropyl acetate/4.0 | no solid was precipitated after the anti-solvent was added dropwise and the solution was stirred under a low temperature, and a solid was precipitated after the solution was transferred to room temperature for volatilization in open for about two days | Crystal Form A |
| 4 | 15.2 | dimethyl sulfoxide/0.1 | isopropyl acetate/4.0 | no solid was precipitated after the anti-solvent was added dropwise, and a solid was precipitated after the solution was transferred to room temperature for volatilization in open for about three days | Crystal form A |
| 5 | 14.8 | dimethylacetami de/0.1 | methyl tert-butyl ether/4.0 | no solid was precipitated after the anti-solvent was added dropwise and the solution was stirred under a low temperature, and a solid was precipitated after the solution was transferred to room temperature for volatilization in open for about two days | Crystal Form A |
| 6 | 15.3 | methyl pyrrolidone/0.1 | pure water/1.0 | a solid was precipitated immediately after the anti-solvent was added dropwise | Crystal Form A |

### Method 2: Reverse anti-solvent addition method

Each about 15 mg of the compound of Formula (I) was weighted and placed into a 3 mL glass vial, and a corresponding volume of positive solvent was added to give a clear solution. The corresponding anti-solvent was added into a 5 mL glass vial and magnetically stirred (rotational speed of about 1000 revolutions per minute), and then the sample solution was added dropwise. No solid was precipitated after the dropwise addition was completed. The sample was transferred to a room temperature condition and volatilized in open for about two days, and then a solid was precipitated. The resulting solid was separated, dried and then tested for XRPD. The experimental results were as follows.

| Test No. | Solid Mass (mg) | Positive Solvent/ Volume (mL) | Anti-solvent/ Volume (mL) | Crystal Form |
|---|---|---|---|---|
| 1 | 15.1 | dimethylformamide/ 0.1 | methyl tert-butyl ether/4.0 | Crystal Form A |
| 2 | 14.7 | acetone/0.4 | 2-butanol/n-heptane (v/v, 3: 1)/2.0 | Crystal Form A |

### Method 3: Gas-liquid permeation method

Each about 15 mg of the compound of Formula (I) was weighed and placed into a 3 mL glass vial, and a corresponding volume of positive solvent was added to dissolve the sample. The sample solution was filtered into a new 3 mL glass vial using a polytetrafluoroethylene filter membrane with 0.45 micron pore size, and the 3 mL glass vial was placed in open into a 20 mL glass vial pre-filled with 4 mL of the corresponding anti-solvent. After the 20 mL glass vial was sealed, it was placed at room temperature to perform gas-liquid permeation until a solid was precipitated. If the solution remained clear after six days, the sample was transferred to a room temperature condition and volatilized in open until a solid was precipitated. The resulting solid was separated, dried and then tested for XRPD. The experimental results were as follows.

| Test No. | Solid Mass (mg) | Positive Solvent/ Volume (mL) | Anti-solvent | Phenomenon | Crystal Form |
|---|---|---|---|---|---|
| 1 | 15.5 | methanol/0.8 | methyl tert-butyl ether | a solid was precipitated after volatilization in open for about five days at room temperature | Crystal Form A |
| 2 | 14.6 | methyl isobutyl ketone/1.0 | n-amyl alcohol | a solid was precipitated after volatilization in open for about five days at room temperature | Crystal Form A |
| 3 | 15.1 | 1,4-dioxane/0.4 | pure water | a solid was precipitated immediately upon gas-liquid permeation | Crystal Form A |
| 4 | 15.4 | acetonitrile/0. 8 | n-hexane | a solid was precipitated after volatilization in open for about five days at room temperature | Crystal form A |

### Method 4: Slow volatilization method

Each about 15 mg of the compound of Formula (I) was weighed and placed into a 3 mL glass vial, and a corresponding volume of positive solvent was added to dissolve the solid. The sample solution was filtered into a new 3 mL glass vial using a polytetrafluoroethylene filter membrane with 0.45 micron pore size. After the glass vial was sealed with a sealing film, four small holes were punched thereon. After the glass vial was placed at room temperature and the solution therein was slowly volatilized for about three days, a solid was precipitated. The resulting solid was separated, dried and then tested for XRPD. The experimental results were as follows.

| Test No. | Solid Mass (mg) | Solvent/Volume (mL) | Crystal Form |
|---|---|---|---|
| 1 | 15.5 | ethyl acetate/1.5 | Crystal Form A |
| 2 | 14.9 | dichloromethane/n-hexane (v/v, 2:1)/1.5 | Crystal Form A |

### Method 5: Gas-solid permeation method

Each about 10 mg of the compound of Formula (I) was weighed and placed into a 3 mL glass vial, which was placed in open into a 20 mL glass vial pre-filled with 4 mL of the corresponding solvent. After the 20 mL glass vial was sealed, it was placed at room temperature to perform gas-solid permeation for about five days. If the solid was completely dissolved, the sample was transferred to a room temperature condition and volatilized in open until a solid was reprecipitated. The resulting solid was separated, dried and then tested for XRPD. The experimental results were as follows.

| Test No. | Solid Mass (mg) | Solvent | Phenomenon | Crystal Form |
|---|---|---|---|---|
| 1 | 9.7 | chloroform | a solid was reprecipitated after volatilization in open for about two days at room temperature | Crystal Form A |
| 2 | 9.6 | toluene | / | Crystal Form A |
| 3 | 10.4 | pure water | / | Crystal Form A |

### Method 6: Slow cooling method

Each about 15 mg of the compound of Formula (I) was weighed and placed into a 1.5 mL glass vial, and 0.4 mL of the corresponding solvent was added to give a suspension. After the suspension was magnetically stirred (rotational speed of about 1000 revolutions per minute) at 50°C for about 2 h, the sample solution was filtered into a new 1.5 mL glass vial using a polytetrafluoroethylene filter membrane with 0.45 micron pore size while the solution is hot. After the vial was sealed, the temperature was lowered from 50°C to 5°C at a rate of 0. 1°C/min, and no solid was precipitated at a constant temperature of 5°C for about two days. The sample was transferred to room temperature condition and volatilized in open until a solid was precipitated. The resulting solid was separated, dried and then tested for XRPD. The experimental results were as follows.

| Test No. | Solid Mass (mg) | Solvent | Phenomenon | Crystal Form |
|---|---|---|---|---|
| 1 | 14.9 | 1-butanol | a solid was precipitated after volatilization in open for about eight days at room temperature | Crystal Form A |
| 2 | 16.1 | methyl ethyl ketone/methyl tert-butyl ether (v/v, 1: 2) | a solid was precipitated after volatilization in open for about two days at room temperature | Crystal Form A |

### Method 7: Suspension stirring method

About 15.7 mg of the compound of Formula (I) was weighed and placed into a 1.5 mL glass vial, and 0.4 mL of a mixed solvent (chloroform/n-heptane, v/v, 1: 2) was added to give a suspension. After the suspension was magnetically stirred (rotational speed of about 1000 revolutions per minute) at 50°C for about 2 h, the solid was obtained by centrifugal separation. The resulting solid was separated, dried and then tested for XRPD, which was the crystal form A.

15.1 mg of the compound of Formula (I) was weighed and placed into a 1.5 mL glass vial, and 0.4 mL of pure water was added to give a suspension, and the suspension was magnetically stirred (rotational speed of about 1000 revolutions per minute) for 6 days. The suspension was centrifuged and the solid was dried in a vacuum oven at 30°C overnight to give the crystal form A of the compound of Formula (I).

### Example 3: Preparation of Crystal Form B of Compound of Formula (I)

A suitable amount of the crystal form A of the compound of Formula (I) was placed in a capped aluminum crucible, and the solid was heated from room temperature to 180°C at a heating rate of 10°C/min under nitrogen protection of 50 mL/min, and then cooled to room temperature to give the crystal form B of the compound of Formula (I).

### Example 4: Preparation of Crystal Form C of Compound of Formula (I)

### Method 1: Gas-solid permeation method

Each about 10 mg of the compound of Formula (I) was weighed and placed into a 3 mL glass vial, which was placed in open into a 20 mL glass vial pre-filled with 4 mL of the corresponding solvent. After the 20 mL glass vial was sealed, it was placed at room temperature to perform gas-solid permeation for about five days. The resulting solid was separated, dried and then tested for XRPD. The experimental results were as follows.

| Test No. | Solid mass (mg) | Solvent | Crystal Form |
|---|---|---|---|
| 1 | 9.8 | methanol | Crystal Form C |
| 2 | 9.8 | ethyl acetate | Crystal Form C |
| 3 | 9.7 | acetone | Crystal Form C |

### Method 2: Suspension stirring method

An appropriate amount of the compound of Formula (I) was weighed and placed into a 1.5 mL glass vial, and 0.4 mL of the corresponding solvent was added to give a suspension. After the suspension was magnetically stirred (rotational speed of about 1000 revolutions per minute) at the corresponding temperature for a period of time, the solid was obtained by centrifugal separation. The resulting solid was separated, dried and then tested for XRPD. The experimental results were as follows.

| Test No. | Solid Mass (mg) | Solvent | Temperature | Stirring Time | Crystal Form |
|---|---|---|---|---|---|
| 1 | 15.3 | methanol/water (v/v, 1: 3) | Room temperature | Six days | Crystal Form C |
| 2 | 14.9 | 1, 4-dioxane/n-heptane (v/v, 1: 4) | Room temperature | Six days | Crystal Form C |
| 3 | 15.4 | dichloromethane/methyl tert-butyl ether (v/v, 1:9) | Room temperature | Six days | Crystal Form C |
| 4 | 69.9 | anisole | Room temperature | Six days | Crystal Form C |
| 5 | 15.7 | isopropanol | Room temperature | Six days | Crystal Form C |
| 6 | 15.1 | isopropanol/water (v/v, 98:2) | Room temperature | Six days | Crystal Form C |
| 7 | 14.9 | isopropanol/water (v/v, 94:6) | Room temperature | Six days | Crystal Form C |
| 8 | 38.4 | isopropanol/water (v/v, 85:15) | Room temperature | Six days | Crystal Form C |
| 9 | 15.1 | isopropyl acetate | Room temperature | Six days | Crystal Form C |
| 10 | 15.8 | ethanol/water (v/v, 1: 3) | 50 °C | Three days | Crystal Form C |
| 11 | 15.3 | methyl isobutyl ketone/ n-heptane (v/v, 1: 4) | 50 °C | Three days | Crystal Form C |
| 12 | 41.3 | ethyl acetate/n-pentanol (v/v, 1: 3) | 50 °C | Six days | Crystal Form C |
| 13 | 14.7 | acetonitrile/methyl tert-butyl ether (v/v, 1: 5) | 50 °C | Three days | Crystal Form C |
| 14 | 14.8 | tetrahydrofuran/pure water (v/v, 1:9) | 50 °C | Three days | Crystal Form C |
| 15 | 15.5 | chloroform/n-heptane (v/v, 1: 5) | 50 °C | Three days | Crystal form C |
| 16 | 16.1 | methyl ethyl ketone/methyl tert-butyl ether (v/v, 1: 2) | 50 °C | Two hours | Crystal Form C |

### Method 3: Slow cooling method

Each about 15.2 mg of the compound of Formula (I) was weighed and placed into a 1.5 mL glass vial, and 0.4 mL of isopropyl acetate was added to give a suspension. After the suspension was magnetically stirred (rotational speed of about 1000 revolutions per minute) at 50°C for about 2 h, the sample solution was filtered into a new 1.5 mL glass vial using a polytetrafluoroethylene filter membrane with 0.45 micron pore size while the solution is hot. After the vial was sealed, the temperature was lowered from 50°C to 5°C at a rate of 0. 1°C/min, and no solid was precipitated at a constant temperature of 5°C for about two days. After the sample solution was transferred to room temperature condition and volatilized in open for about two days, a solid was precipitated. The resulting solid was separated, dried and then tested for XRPD, which was crystal form C.

### Example 5: Single Crystal X-ray Diffraction Detection and Analysis of Compound of Formula (I)

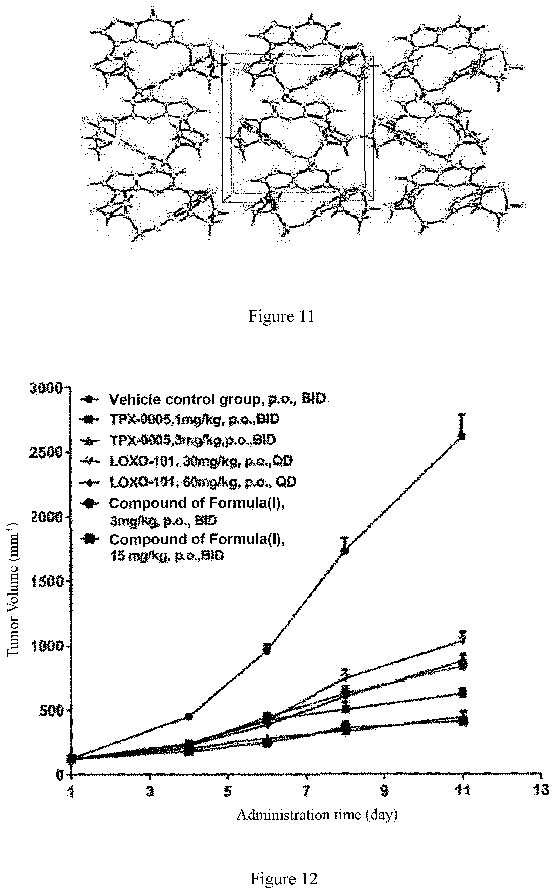

Cultivation Processes of Single Crystal: About 5 mg of the sample was taken and placed into a 2 mL brown sample bottle, and 400 µL acetone was added. After the sample was dissolved sufficiently, the resulting mixture was allowed to stand at room temperature. After 15 days, small pieces of colorless crystals appeared. The crystals were collected and the diffraction intensity data were collected by Bruker D8 venture diffractometer. The absolute configuration of the compound of Formula (I) can be determined from the single crystal data of the compound. An ellipsoid diagram of the stereostructure of the compound of Formula (I) is shown in Figure 10. The unit cell stacking diagram of the compound of Formula (I) along the b-axis direction is shown in Figure 11. The crystal structure data and parameters of the compound of Formula (I) are shown in Tables 4, 5, 6, 7 and 8.

**Table 4. Crystal Data and Structural Particulars of Compound of Formula (I)**

| | |
|---|---|
| Identification code | Compound of Formula (I) |
| Empirical Formula | C₁₉ H₁₇ F N₆ O₃ |
| Formula weight | 396.38 |
| Temperature | 296(2) K |
| Wavelength | 1.54178 A |
| Crystal system, space group | Monoclinic, P2(1) |
| Unit cell dimensions | a = 9.459(2) A alpha = 90 deg. |
| | b = 9.5561(18) A beta = 104.284(12) deg |
| | c = 10.2634(15) A gamma = 90 deg |
| Volume | 899.0(3) A^3 |
| Z, Calculated density | 2, 1.464 Mg/m^3 |
| Absorption coefficient | 0.927 mm^-1 |
| F(000) | 412 |
| Crystal size | 0.14 x 0.12 x 0.10 mm |
| Theta range for data collection | 4.824 to 68.220 deg. |
| Limiting indices | -10<=h<= 11, -11 <=k<= 11, -12<=1<=12 |
| Reflections collected / unique | 5706 / 2970 [R(int) = 0.0540] |
| Completeness to theta = 67.679 | 97.5 % |
| Absorption correction | None |
| Refinement method | Full-matrix least-squares on F^2 |
| Data / restraints / parameters | 2970 / 1 / 262 |
| Goodness-of-fit on F^2 | 1.172 |
| Final R indices [I>2sigma(I)] | R1 = 0.0631, wR2 = 0.1967 |
| R indices (all data) | R1 = 0.1820, wR2 = 0.2762 |
| Absolute structure parameter | 0.08(9) |
| Extinction coefficient | n/a |
| Largest diff peak and hole | 0.522 and -0.630 e.A^-3 |

**Table 5. Atomic Coordinates (×10⁴) and Equivalent Isotropic Shift Parameters (Å²×10³) of Crystals of Compound of Formula (I)**

| | x | y | z | U(eq) |
|---|---|---|---|---|
| F(1) | 6812(7) | 4407(7) | 1056(5) | 81(2) |
| O(1) | 3449(10) | 5680(10) | 8512(6) | 87(3) |
| O(2) | 607(8) | 4237(6) | 846(5) | 61(2) |
| O(3) | 4210(7) | 6941(5) | 4537(4) | 49(2) |
| N(1) | 4229(9) | 5117(7) | 6669(6) | 59(2) |
| N(2) | -81(12) | 2900(8) | 6615(8) | 69(3) |
| N(3) | 160(9) | 3156(6) | 5368(6) | 51(2) |
| N(4) | 1851(8) | 4365(5) | 4387(5) | 42(2) |
| N(5) | 1592(9) | 4394(6) | 2127(5) | 50(2) |
| N(6) | 6319(11) | 6271(6) | 3951(7) | 55(2) |
| C(1) | 3257(11) | 5076(7) | 7438(6) | 48(2) |
| C(2) | 1940(11) | 4234(6) | 6826(7) | 52(2) |
| C(3) | 964(12) | 3587(8) | 7455(9) | 58(3) |
| C(4) | -596(12) | 2711(7) | 4183(9) | 57(2) |
| C(5) | -176(13) | 3074(8) | 3050(8) | 56(2) |
| C(6) | 1055(10) | 3958(6) | 3207(7) | 47(2) |
| C(7) | 1406(10) | 3949(6) | 5477(7) | 42(2) |
| C(8) | 2318(10) | 5803(7) | 2215(6) | 46(2) |
| C(9) | 1556(12) | 6472(9) | 855(7) | 58(3) |
| C(10) | 154(12) | 5636(10) | 448(7) | 63(3) |
| C(11) | 3969(10) | 5714(7) | 2504(6) | 45(2) |
| C(12) | 4627(13) | 5065(8) | 1605(7) | 57(3) |
| C(13) | 6144(13) | 5048(8) | 1921(8) | 58(3) |
| C(14) | 6933(12) | 5656(8) | 3050(8) | 57(2) |
| C(15) | 4873(12) | 6291(6) | 3667(7) | 49(2) |
| C(16) | 5077(12) | 7309(7) | 5865(7) | 54(2) |
| C(17) | 5436(13) | 6045(8) | 6774(7) | 57(3) |
| C(18) | 6499(13) | 6198(10) | 8125(7) | 67(3) |
| C(19) | 6938(14) | 5373(10) | 7029(10) | 73(3) |
| H(1A) | 4104 | 4506 | 6036 | 70 |
| H(3A) | 1044 | 3636 | 8375 | 69 |
| H(4B) | -1414 | 2151 | 4121 | 68 |
| H(5A) | -678 | 2754 | 2207 | 68 |
| H(8A) | 2059 | 6340 | 2937 | 55 |
| H(9A) | 1360 | 7457 | 957 | 70 |
| H(9B) | 2138 | 6368 | 204 | 70 |
| H(10A) | -559 | 5967 | 914 | 75 |
| H(10B) | -263 | 5692 | -514 | 75 |
| H(12A) | 4078 | 4656 | 821 | 68 |
| H(14A) | 7945 | 5650 | 3213 | 69 |
| H(16A) | 4546 | 7983 | 6267 | 64 |
| H(16B) | 5977 | 7747 | 5785 | 64 |
| H(18A) | 6302 | 5680 | 8873 | 80 |
| H(18B) | 6948 | 7106 | 8357 | 80 |
| H(19A) | 6998 | 4363 | 7120 | 88 |
| H(19B) | 7645 | 5790 | 6603 | 88 |

**Table 6. Bond Length (Å) and Bond Angle[deg] of Compound of Formula (I)**

| | | | |
|---|---|---|---|
| F(1)-C(13) | 1.356(9) | C(5)-H(5A) | 0.9300 |
| O(1)-C(1) | 1.218(9) | C(8)-C(11) | 1.518(13) |
| O(2)-N(5) | 1.420(8) | C(8)-C(9) | 1.542(10) |
| O(2)-C(10) | 1.432(11) | C(8)-H(8A) | 0.9800 |
| O(3)-C(15) | 1.361(8) | C(9)-C(10) | 1.516(15) |
| O(3)-C(16) | 1.450(9) | C(9)-H(9A) | 0.9700 |
| N(1)-C(1) | 1.352(10) | C(9)-H(9B) | 0.9700 |
| N(1)-C(17) | 1.429(11) | C(10)-H(10A) | 0.9700 |
| N(1)-H(1A) | 0.8600 | C(10)-H(10B) | 0.9700 |
| N(2)-C(3) | 1.315(13) | C(11)-C(12) | 1.381(9) |
| N(2)-N(3) | 1.376(9) | C(11)-C(15) | 1.398(12) |
| N(3)-C(4) | 1.320(11) | C(12)-C(13) | 1.390(15) |
| N(3)-C(7) | 1.382(11) | C(12)-H(12A) | 0.9300 |
| N(4)-C(6) | 1.317(9) | C(13)-C(14) | 1.346(13) |
| N(4)-C(7) | 1.349(8) | C(14)-H(14A) | 0.9300 |
| N(5)-C(6) | 1.392(8) | C(16)-C(17) | 1.512(10) |
| N(5)-C(8) | 1.504(10) | C(16)-H(16A) | 0.9700 |
| N(6)-C(15) | 1.326(13) | C(16)-H(16B) | 0.9700 |
| N(6)-C(14) | 1.343(10) | C(17)-C(18) | 1.505(12) |
| C(1)-C(2) | 1.486(13) | C(17)-C(19) | 1.522(15) |
| C(2)-C(7) | 1.379(10) | C(18)-C(19) | 1.513(13) |
| C(2)-C(3) | 1.395(10) | C(18)-H(18A) | 0.9700 |
| C(3)-H(3A) | 0.9300 | C(18)-H(18B) | 0.9700 |
| C(4)-C(5) | 1.363(11) | C(19)-H(19A) | 0.9700 |
| C(4)-H(4B) | 0.9300 | C(19)-H(19B) | 0.9700 |
| C(5)-C(6) | 1.415(13) | | |
| N(5)-O(2)-C(10) | 104.3(5) | H(9A)-C(9)-H(9B) | 109.2 |
| C(15)-O(3)-C(16) | 118.8(7) | O(2)-C(10)-C(9) | 103.5(8) |
| C(1)-N(1)-C(17) | 128.3(6) | O(2)-C(10)-H(10A) | 111.1 |
| C(1)-N(1)-H(1A) | 115.8 | C(9)-C(10)-H(10A) | 111.1 |
| C(17)-N(1)-H(1A) | 115.8 | O(2)-C(10)-H(10B) | 111.1 |
| C(3)-N(2)-N(3) | 104.5(7) | C(9)-C(10)-H(10B) | 111.1 |
| C(4)-N(3)-N(2) | 128.6(7) | H(10A)-C(10)-H(10B) | 109.0 |
| C(4)-N(3)-C(7) | 120.6(6) | C(12)-C(11)-C(15) | 117.7(9) |
| N(2)-N(3)-C(7) | 110.8(7) | C(12)-C(11)-C(8) | 120.4(7) |
| C(6)-N(4)-C(7) | 116.8(7) | C(15)-C(11)-C(8) | 121.8(6) |
| C(6)-N(5)-O(2) | 114.9(7) | C(11)-C(12)-C(13) | 117.3(8) |
| C(6)-N(5)-C(8) | 118.1(5) | C(11)-C(12)-H(12A) | 121.3 |
| O(2)-N(5)-C(8) | 109.6(5) | C(13)-C(12)-H(12A) | 121.3 |
| C(15)-N(6)-C(14) | 117.0(8) | C(14)-C(13)-F(1) | 120.6(10) |
| O(1)-C(1)-N(1) | 122.9(8) | C(14)-C(13)-C(12) | 121.1(7) |
| O(1)-C(1)-C(2) | 124.2(8) | F(1)-C(13)-C(12) | 118.2(8) |
| N(1)-C(1)-C(2) | 112.9(6) | N(6)-C(14)-C(13) | 122.6(10) |
| C(7)-C(2)-C(3) | 104.9(8) | N(6)-C(14)-H(14A) | 118.7 |
| C(7)-C(2)-C(1) | 126.3(6) | C(13)-C(14)-H(14A) | 118.7 |
| C(3)-C(2)-C(1) | 128.8(7) | N(6)-C(15)-O(3) | 118.7(8) |
| N(2)-C(3)-C(2) | 113.2(7) | N(6)-C(15)-C(11) | 124.2(7) |
| N(2)-C(3)-H(3A) | 123.4 | O(3)-C(15)-C(11) | 117.1(9) |
| C(2)-C(3)-H(3A) | 123.4 | O(3)-C(16)-C(17) | 112.0(6) |
| N(3)-C(4)-C(5) | 119.9(8) | O(3)-C(16)-H(16A) | 109.2 |
| N(3)-C(4)-H(4B) | 120.1 | C(17)-C(16)-H(16A) | 109.2 |
| C(5)-C(4)-H(4B) | 120.1 | O(3)-C(16)-H(16B) | 109.2 |
| C(4)-C(5)-C(6) | 117.4(8) | C(17)-C(16)-H(16B) | 109.2 |
| C(4)-C(5)-H(5A) | 121.3 | H(16A)-C(16)-H(16B) | 107.9 |
| C(6)-C(5)-H(5A) | 121.3 | N(1)-C(17)-C(18) | 118.0(6) |
| N(4)-C(6)-N(5) | 113.9(7) | N(1)-C(17)-C(16) | 113.3(9) |
| N(4)-C(6)-C(5) | 123.3(6) | C(18)-C(17)-C(16) | 119.2(7) |
| N(5)-C(6)-C(5) | 122.7(7) | N(1)-C(17)-C(19) | 116.5(7) |
| N(4)-C(7)-C(2) | 131.5(8) | C(18)-C(17)-C(19) | 60.0(7) |
| N(4)-C(7)-N(3) | 121.9(7) | C(16)-C(17)-C(19) | 120.1(7) |
| C(2)-C(7)-N(3) | 106.5(6) | C(17)-C(18)-C(19) | 60.6(6) |
| N(5)-C(8)-C(11) | 113.2(6) | C(17)-C(18)-H(18A) | 117.7 |
| N(5)-C(8)-C(9) | 102.2(7) | C(19)-C(18)-H(18A) | 117.7 |
| C(11)-C(8)-C(9) | 115.4(6) | C(17)-C(18)-H(18B) | 117.7 |
| N(5)-C(8)-H(8A) | 108.6 | C(19)-C(18)-H(18B) | 117.7 |
| C(11)-C(8)-H(8A) | 108.6 | H(18A)-C(18)-H(18B) | 114.8 |
| C(9)-C(8)-H(8A) | 108.6 | C(18)-C(19)-C(17) | 59.5(6) |
| C(10)-C(9)-C(8) | 102.3(7) | C(18)-C(19)-H(19A) | 117.8 |
| C(10)-C(9)-H(9A) | 111.3 | C(17)-C(19)-H(19A) | 117.8 |
| C(8)-C(9)-H(9A) | 111.3 | C(18)-C(19)-H(19B) | 117.8 |
| C(10)-C(9)-H(9B) | 111.3 | C(17)-C(19)-H(19B) | 117.8 |
| C(8)-C(9)-H(9B) | 111.3 | H(19A)-C(19)-H(19B) | 115.0 |

**Table 7. Torsional Angle [deg] of Compound of Formula (I)**

| | | | |
|---|---|---|---|
| C(3)-N(2)-N(3)-C(4) | -179.4(8) | C(6)-N(5)-C(8)-C(9) | -130.8(8) |
| C(3)-N(2)-N(3)-C(7) | 2.9(9) | O(2)-N(5)-C(8)-C(9) | 3.3(7) |
| C(10)-O(2)-N(5)-C(6) | 107.2(7) | N(5)-C(8)-C(9)-C(10) | 21.5(7) |
| C(10)-O(2)-N(5)-C(8) | -28.6(8) | C(11)-C(8)-C(9)-C(10) | 144.7(6) |
| C(17)-N(1)-C(1)-O(1) | -14.5(15) | N(5)-O(2)-C(10)-C(9) | 42.2(7) |
| C(17)-N(1)-C(1)-C(2) | 165.0(8) | C(8)-C(9)-C(10)-O(2) | -39.4(6) |
| O(1)-C(1)-C(2)-C(7) | 155.4(9) | N(5)-C(8)-C(11)-C(12) | 62.4(8) |
| N(1)-C(1)-C(2)-C(7) | -24.1(11) | C(9)-C(8)-C(11)-C(12) | -54.9(9) |
| O(1)-C(1)-C(2)-C(3) | -23.2(13) | N(5)-C(8)-C(11)-C(15) | -118.3(6) |
| N(1)-C(1)-C(2)-C(3) | 157.3(8) | C(9)-C(8)-C(11)-C(15) | 124.4(7) |
| N(3)-N(2)-C(3)-C(2) | -3.1(10) | C(15)-C(11)-C(12)-C(13) | -0.6(10) |
| C(7)-C(2)-C(3)-N(2) | 2.1(10) | C(8)-C(11)-C(12)-C(13) | 178.7(6) |
| C(1)-C(2)-C(3)-N(2) | -179.1(8) | C(11)-C(12)-C(13)-C(14) | -0.8(11) |
| N(2)-N(3)-C(4)-C(5) | -179.7(8) | C(11)-C(12)-C(13)-F(1) | 179.6(6) |
| C(7)-N(3)-C(4)-C(5) | -2.2(12) | C(15)-N(6)-C(14)-C(13) | -1.7(11) |
| N(3)-C(4)-C(5)-C(6) | -1.6(13) | F(1)-C(13)-C(14)-N(6) | -178.3(6) |
| C(7)-N(4)-C(6)-N(5) | -178.5(6) | C(12)-C(13)-C(14)-N(6) | 2.1(12) |
| C(7)-N(4)-C(6)-C(5) | -2.9(11) | C(14)-N(6)-C(15)-O(3) | -178.1(5) |
| O(2)-N(5)-C(6)-N(4) | -167.0(6) | C(14)-N(6)-C(15)-C(11) | 0.2(9) |
| C(8)-N(5)-C(6)-N(4) | -35.2(10) | C(16)-O(3)-C(15)-N(6) | -13.5(8) |
| O(2)-N(5)-C(6)-C(5) | 17.3(10) | C(16)-O(3)-C(15)-C(11) | 168.0(5) |
| C(8)-N(5)-C(6)-C(5) | 149.1(8) | C(12)-C(11)-C(15)-N(6) | 1.0(9) |
| C(4)-C(5)-C(6)-N(4) | 4.4(12) | C(8)-C(11)-C(15)-N(6) | -178.4(6) |
| C(4)-C(5)-C(6)-N(5) | 179.6(8) | C(12)-C(11)-C(15)-O(3) | 179.3(6) |
| C(6)-N(4)-C(7)-C(2) | -176.9(7) | C(8)-C(11)-C(15)-O(3) | 0.0(8) |
| C(6)-N(4)-C(7)-N(3) | -1.2(10) | C(15)-O(3)-C(16)-C(17) | -75.0(8) |
| C(3)-C(2)-C(7)-N(4) | 176.0(8) | C(1)-N(1)-C(17)-C(18) | 51.7(14) |
| C(1)-C(2)-C(7)-N(4) | -2.9(14) | C(1)-N(1)-C(17)-C(16) | -94.5(10) |
| C(3)-C(2)-C(7)-N(3) | -0.2(8) | C(1)-N(1)-C(17)-C(19) | 120.0(10) |
| C(1)-C(2)-C(7)-N(3) | -179.0(7) | O(3)-C(16)-C(17)-N(1) | -42.7(9) |
| C(4)-N(3)-C(7)-N(4) | 3.8(11) | O(3)-C(16)-C(17)-C(18) | 171.6(8) |
| N(2)-N(3)-C(7)-N(4) | -178.3(7) | O(3)-C(16)-C(17)-C(19) | 101.4(9) |
| C(4)-N(3)-C(7)-C(2) | -179.6(7) | N(1)-C(17)-C(18)-C(19) | 106.0(9) |
| N(2)-N(3)-C(7)-C(2) | -1.7(9) | C(16)-C(17)-C(18)-C(19) | -109.9(9) |
| C(6)-N(5)-C(8)-C(11) | 104.5(8) | N(1)-C(17)-C(19)-C(18) | -108.5(7) |
| O(2)-N(5)-C(8)-C(11) | -121.4(6) | C(16)-C(17)-C(19)-C(18) | 108.5(8) |

**Table 8. Hydrogen Bond [A and deg] of Compound of Formula (I)**

| D-H...A | d(D-H) | (H...A) | d(D...A) | <(DHA) |
|---|---|---|---|---|
| N(1)-H(1A)...N(4) | 0.86 | 2.38 | 2.908(10) | 120.6 |

### Example 6: Solid Stability Test of Crystal Form C of Compound of Formula (I)

The crystal form C of the compound of Formula (I) was weighed and placed in the bottom of a glass sample bottle to form a thin layer. The sample placed under a high temperature condition was sealed and left to stand in a stability test chamber at 60°C. After 10 days, the sample was taken and tested for solid purity and crystal form. The sample placed under a high humidity condition was left to stand at room temperature and 92.5% RH humidity (the mouth of the sample bottle was wrapped with a layer of sealing film and 15~20 small holes were evenly punched in the sealing film), and the samples were taken and tested for solid purity and crystal form after 5 days/10 days, respectively. The experimental results were shown in Table 9 below.

**Table 9. Summary of Experimental Results of Evaluation of Influence Factors for Crystal Form C of Compound of Formula (I)**

| Test Conditions | Sampling Time | Initial Purity | Test Results | | |
|---|---|---|---|---|---|
| | | | Purity | Relative Purity^{∗} | Crystal Form |
| 60°C | 10 days | 100.0% | 100.0% | 100.0% | Crystal Form C |
| Room temperature/ 92.5%RH | 5 days | | 100.0% | 100.0% | Crystal Form C |
| | 10 days | | 100.0% | 100.0% | Crystal Form C |

| | | | | | |
|---|---|---|---|---|---|
| Note: Peaks with areas less than 0.05% were not integrated; and ^{∗} indicates that relative purity = purity of stability sample/ purity of starting sample×100% | | | | | |

The crystal form C of the compound of Formula (I) was weighed and left to stand in 40°C/75%RH and 60°C/75%RH stability test chambers (the mouth of the sample bottle was wrapped with a layer of sealing film and 15~20 small holes were evenly punched in the sealing film). Samples were respectively taken at 1 month, 2 months and 3 months to test the solid chemical purity (HPLC area purity) and crystal form. The experimental results were shown in Table 10 below.

**Table 10: Summary of Evaluation of Accelerated Stability Test**

| Sampling Point | 40°C/75% RH Condition | | | 60°C/75%RH Condition | | |
|---|---|---|---|---|---|---|
| | Purity | Relative Purity^{∗} | Crystal Form | Purity | Relative Purity^{∗} | Crystal Form |
| Initial | 100.0 | / | / | 100.0 | / | / |
| 1 month | 100.0 | 100.0 | Crystal Form C | 99.9 | 99.9 | Crystal Form C |
| 2 months | 100.0 | 100.0 | Crystal Form C | 100.0 | 100.0 | Crystal Form C |
| 3 months | 100.0 | 100.0 | Crystal Form C | 100.0 | 100.0 | Crystal Form C |

| | | | | | | |
|---|---|---|---|---|---|---|
| Area reject: 0.05% | | | | | | |

Conclusion: The compound of Formula (I) has good stability under high temperature, high humidity and accelerated conditions, and is easy for preparation into drugs.

### Experimental Example 1: Inhibitory Activities of Compounds against Kinases such as TrkA, TrkC, ALK, and Ros1

The inhibitory activity tests of compounds against kinases such as TrkA, TrkC, ALK, Ros1 were completed in Reaction Biology Corp.. Certain concentrations of substrate, cofactors, kinases and test compounds (10 doses, 3-fold serial dilutions, 2%DMSO final concentration) were sequentially added to the reaction buffer (20 mM Hepes (pH 7.5), 10 mM MgCl₂, 1 mM EGTA, 0.02% Brij35, 0.02 mg/mL BSA, 0.1 mM Na₃VO₄, 2 mM DTT, 1% DMSO), and mixed well. The resulting mixture was incubated at room temperature for 20 minutes, and a certain concentration of 33P-ATP was added to the reaction mixture to initiate the reaction, followed by incubation at room temperature for 120 minutes. Finally, the radioactivity of the reactants was detected by a filtration-binding method. The final kinase activity was expressed by the ratio of the remaining kinase activity in the test sample to the kinase activity of the DMSO control group. The dose-effect curve was fitted by GraphPad software and the IC₅₀ values were calculated. The results were shown in Table 11.

**Table 11: Half Inhibitory Concentrations of Kinases IC₅₀ (nM)**

| Compound No. | TrkA | TrkA-G595R | TrkA-G667C | TrkC | ALK | Ros1 | Ros-G2032R |
|---|---|---|---|---|---|---|---|
| Compound of Formula (I) | 2.07 | 3.60 | 7.00 | 0.14 | 13.00 | 0.14 | 0.4 |
| LOXO-101 | 19.70 | >1000 | 512.00 | 1.12 | >1000 | 95.50 | / |
| LOXO-195 | 6.67 | 6.19 | 110.00 | 0.50 | 274.00 | 1.15 | 2.88 |

| | | | | | | | |
|---|---|---|---|---|---|---|---|
| "/" means not detected. | | | | | | | |

Conclusion: The compound of Formula (I) of the present invention exhibits higher kinase inhibitory activities in a variety of kinases and mutants thereof, and shows strong inhibitions on mutations in the gate-keeping group region (gatekeeper), the solvent front region (solvent front mutation) and the DFG region of a variety of kinases.

### Experimental Example 2: Inhibitory Activities of Compounds on Cell Proliferation

Adenosine Tri-Phosphate (ATP) is an energy carrier shared by various life activities in nature and is the smallest unit of energy storage and transfer. In CellTiter-Glo^{™} Live Cell Detection Kit, luciferase was used as a detection substance, and it required the involvement of ATP during luminescence. CellTiter-Glo^{™} reagent was added to the cell culture medium to measure a luminescence value, and the light signal was proportional to the amount of ATP in the system, and ATP was positively correlated to the number of viable cells. Therefore, by detecting the ATP content using the CellTiter-Glo Kit, cell proliferation can be detected. In this test, the cell line was Ba/F3 LMNA-NTRK1-WT stable transformant cell line, and the number of cells was 5000/well.

### IC₅₀ Assay Procedure:

### 1. Cell Culture and Inoculation

a) Cells in the logarithmic growth phase were harvested and counted by using a platelet counter. Cell viability was detected by trypan blue exclusion assay to ensure that the cell viability was above 90%.
b) The cell concentration was adjusted, and 90 µL cell suspension was added to a 96-well plate, respectively.
c) Cells in the 96-well plate were incubated overnight at 37°C, 5% CO₂ and 95% humidity.

### 2. Drug Dilution and Dosing

a) A 10-fold drug solution was prepared with a maximum concentration of 10 µM at 9 concentrations and 3-fold dilution (refer to Appendix 1). 10 µL of the drug solution was added to each well in a 96-well plate seeded with cells, with three replicate wells for each drug concentration.
b) The cells in the 96-well plate added with the drug were incubated for a further 72 h at 37°C, 5% CO₂ and 95% humidity and then subjected to CTG (cell proliferation profile) assay.

### 3. Endpoint Plate Reading

a) CellTiter-Glo^{™} reagent was thawed and the cell plate was equilibrated to room temperature for 30 minutes.
b) An equal volume of CellTiter-Glo^{™} reagent was added to each well.
c) Cells were lysed by shaking on an orbital shaker for 5 minutes.
d) The cell plate was left at room temperature for 20 minutes to stabilize the luminescence signal.
e) The luminescence values were read.

### 4. Data Processing

The data were analyzed using GraphPad Prism 5.0 software, and were fitted to obtain a dose-effect curve using a non-linear S-curve regression, and the IC₅₀ values were calculated therefrom. The data were shown in Table 12.

**Table 12. Half Inhibitory Concentrations of Cells IC₅₀ (nM)**

| Compound No. | Ba/F3 LMNA-NTRK1-WT | Ba/F3 LMNA-NTRK1-F589L | Ba/F3 LMNA-NTRK1-G595R | BaF3 ETV6-NTRK3-G623R | Ba/F3 SLC34A2-ROS1-WT | Ba/F3 SLC34A2-ROS1-G2032R |
|---|---|---|---|---|---|---|
| Compound of Formula (I) | 1.26 | 0.78 | 6.20 | 3.40 | 2.53 | 16.08 |

Conclusion: The compound of Formula (I) of the present invention exhibits a higher cell proliferation inhibitory activity against Ba/F3 LMNA-NTRK1-WT stable transgenic cell line. Meanwhile, the compound of Formula (I) of the present invention shows higher cell proliferation inhibitory activities against Ba/F3 LMNA-NTRK1-F589L, Ba/F3 LMNA-NTRK1-G595R, BaF3 ETV6-NTRK3-G623R, Ba/F3 SLC34A2-ROS1-WT and Ba/F3 SLC34A2-ROS1-G2032R stable transgenic cell lines.

### Experimental Example 3: Cassette Pharmacokinetic Test of Compounds in Mice

**Experimental Objective:** The drug concentrations of the compound of Formula (I), TPX0005, Entrectinib (RXDX-101) and Larotrectinib (LOXO-101) in plasma and specific tissues at different time points were determined by LC/MS/MS method after single intravenous (IV) and intragastric (PO) cassette administration of the compound of Formula (I), TPX0005, Entrectinib (RXDX-101) and Larotrectinib (LOXO-101) to 7-9 week old male CD-1 mice as the test animals. The pharmacokinetic behaviors of the compounds of the present invention in mice were studied and the pharmacokinetic properties thereof were evaluated.

**Pharmaceutical Preparation:** The compound of Formula (I), TPX0005, Entrectinib (RXDX-101) and Larotrectinib (LOXO-101) were all prepared into clear solutions using 5%DMSO+10%solutol+85% water as a solvent for administration in IV (intravenous injection) and PO (gavage) groups. Each compound was administered at a dosage of 1 mg/kg and a dose volume of 2 mL/kg for the IV group, and at a dosage of 3 mg/kg and a dose volume of 3 mL/kg for PO group.

The results of pharmacokinetic parameters were shown in Table 11.

**Table 11. Test Results of Cassette Pharmacokinetic Test in Mice**

| Compound No. | | LOXO-101 | RXDX-101 | TPX-0005 | Compound of Formula (I) |
|---|---|---|---|---|---|
| IV @ 1 mpk | Initial Concentration C₀ (nM) | 2379 | 2808 | 2253 | 3708 |
| | Half-life T_{1/2} (h) | 1.21 | 1.94 | 3.69 | 0.88 |
| | Apparent Distribution Volume Vd (L/kg) | 1.57 | 1.24 | 2.63 | 0.57 |
| | Apparent Clearance Cl (mL/Kg/min) | 22.7 | 7.9 | 16.5 | 6.3 |
| | Area under Curve AUC_{0-inf} (nM.hr) | 1717 | 3767 | 2857 | 6689 |
| PO @3 mpk | Peak Concentration Cₘₐₓ (nM) | 1353 | 586 | 1540 | 4740 |
| | Time to Peak Tₘₐₓ (h) | 0.5 | 0.75 | 0.75 | 0.25 |
| | Area under Curve AUC_{0-inf} (nM.hr) | 2720 | 3256 | 5494 | 19769 |
| | Bioavailability F% | 51% | 23% | 64% | 76% |
| | Drug Concentration in Brain at 0.5 h (Brain@0.5h (nmol/kg)) | 40 | ND | 50 | 663 |
| | Drug Concentration in Brain at 2 h (Brain@2h (nmol/kg)) | 20 | 30 | 41 | 343 |
| | Drug Concentration in Cerebrospinal Fluid at 0.5 h (CSF@0.5h (nmol/kg)) | 15 | ND | 8 | 63 |
| | Drug Concentration in Cerebrospinal Fluid at 2 h (CSF@2h (nmol/kg)) | 3 | ND | ND | 21 |

| | | | | | |
|---|---|---|---|---|---|
| "Nd" means not detected. | | | | | |

Conclusion: The compound of Formula (I) of the present invention has better pharmacokinetic properties in mice. Where the administration dosages were same, the total systemic exposure of the compound of Formula (I) of the present invention after oral administration, and the exposure of the compound of Formula (I) of the present invention in the brain and cerebrospinal fluid CSF after administration for 0.5 h and 2 h were significantly higher than the corresponding exposures of TPX0005, Entrectinib (RXDX-101) and Larotrectinib (LOXO-101).

### Experimental Example 4: Pharmacokinetic Test of Compounds in Mice

**Experimental Objective:** The drug concentrations of the compounds in plasma at different time points were determined by LC/MS/MS method after single intravenous (IV) and intragastric (PO) administration of the compounds to 7-9 week old male CD-1 mice as the test animals. The pharmacokinetic behaviors of the compounds of the present invention in mice were studied and pharmacokinetic properties thereof were evaluated.

**Pharmaceutical Preparation:** The compounds were prepared into clear solutions using 5%DMSO+10%solutol+85% water as a solvent for administration in IV (intravenous injection) and PO (gavage) groups. The administration dosage of each compound was 3 mg/kg for the IV group and 10 mL/kg for the PO group.

The results of pharmacokinetic parameters were shown in Table 12.

**Table 12. Results of Pharmacokinetic Test in Mice**

| Compound No. | | LOXO-101 | LOXO-195 | Compound of Formula (I) |
|---|---|---|---|---|
| IV | Half-life T_{1/2} (h) | 0.39 | 1.19 | 0.75 |
| | Apparent Distribution Volume Vd (L/kg) | 0.97 | 0.50 | 0.49 |
| | Apparent Clearance Cl (mL/Kg/min) | 37.4 | 11.8 | 7.2 |
| | Area under Curve AUC₀₋ₗₐₛₜ (nM.hr) | 3122 | 11146 | 17502 |
| PO | Peak Concentration Cₘₐₓ (nM) | 1548 | 12700 | 9145 |
| | Time to Peak Tₘₐₓ (h) | 0.38 | 0.38 | 0.50 |
| | Area under Curve AUC₀₋ₗₐₛₜ (nM.hr) | 2890 | 27344 | 29787 |
| | Bioavailability F% | 28% | 74% | 51% |

Conclusion: At the same administration dosage, the total systemic exposure, peak concentration, and bioavailability of the compound of the present invention after oral administration were significantly superior to Larotrectinib (LOXO-101) and LOXO-195, exhibiting excellent pharmacokinetic properties.

### Experimental Example 5: Efficacy Test of Compounds in Mice

**Experimental Objective:** The *in vivo* efficacy of test drugs such as the compound of Formula (I) on human colon cancer cell line KM12 cells subcutaneous xenograft tumor in a BALB/c mouse model was evaluated.

**Drug Preparation:** The compounds were prepared into clear solutions using 5%DMSO+10%solutol+85% water as a solvent for administration in PO (gavage) groups.

**Tumor Measurement:** The tumor diameter was measured twice a week using a vernier caliper. The formula for calculating the tumor volume is: V = 0.5×a × b², wherein a and b represent the long diameter and the short diameter of the tumor, respectively. The antitumor efficacy of the compounds was evaluated with TGI (%), which reflects the inhibition rate of tumor growth. TGI (%) =[(1-(average tumor volume at the end of administration in a treatment group-average tumor volume at the beginning of administration in the treatment group))/(average tumor volume at the end of treatment in a solvent control group-average tumor volume at the beginning of treatment in the solvent control group)] × 100%. The results were shown in Figure 12.

**Statistical Analysis:** Statistical analysis was performed using SPSS software based on relative tumor volume and tumor weight at the end of the experiment. Comparisons among multiple groups were analyzed by one-way ANOVA. If the variances were homogeneous (no significant difference in F-values), the analysis was performed by the Tukey's method, and if the variances were not homogeneous (significant difference in F-values), the test was performed by the Games-Howell method. P < 0.05 was considered as a significant difference.

**Experimental Results:** In the human colon cancer KM12 nude mouse xenograft tumor model, the test compound of Formula (I) showed significant anti-tumor effects at a dosage as low as 3 mg/kg, and the anti-tumor effect tended to be dose-dependent (p < 0.05 between the comparison of the high dose group and the low dose group). The anti-tumor effect (T/C=33.18%, TGI=71.23%) of the compound of Formula (I) at a dosage of 3 mg/kg was comparable (P > 0.05) to the anti-tumor effect (T/C=34.20%, TGI=69.73%) of the high dose group (60 mg/kg) of the compound LOXO-101. The anti-tumor effect (T/C=15.63%, TGI=88.61%) of the compound of Formula (I) at a dosage of 15 mg/kg was superior to the anti-tumor effect (T/C=34.20%, TGI=69.73%) of the high dose group (60 mg/kg) of the compound LOXO-101 and was comparable (P > 0.05) to the anti-tumor effect (T/C=16.80%, TGI=87.46%) of the high dose group (3 mg/kg) of the compound TPX-0005.

### Experimental Example 6: Efficacy Test of Compounds in Mice

**Experimental Objective:** The *in vivo* efficacy of test drugs such as the compound of Formula (I) on human lung cancer LU-01-0414 subcutaneous xenograft tumor in a BALB/c mouse model was evaluated.

**Drug Preparation:** The compounds were prepared into clear solutions using 5%DMSO+10%solutol+85% water as a solvent for administration in PO (gavage) groups.

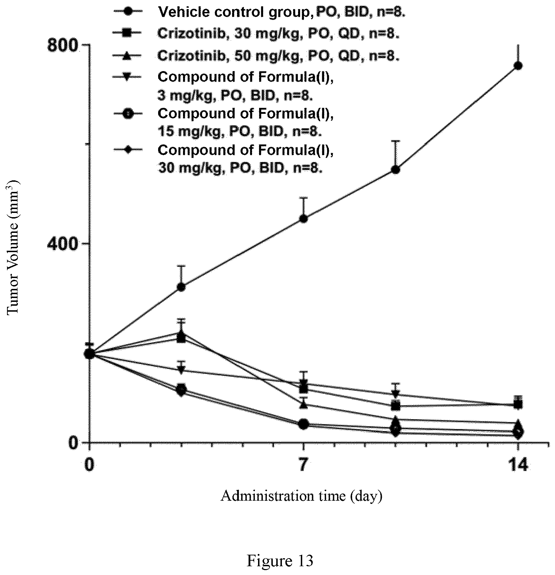

**Tumor Measurement:** The tumor diameters were measured twice a week using a vernier caliper. The formula for calculating the tumor volume is: V = 0.5×a × b², wherein a and b represent the long diameter and the short diameter of the tumor, respectively. The antitumor efficacy of the compounds was evaluated with TGI (%), which reflects the inhibition rate of tumor growth. TGI (%) =[(1-(average tumor volume at the end of administration in a treatment group-average tumor volume at the beginning of administration in the treatment group))/(average tumor volume at the end of treatment in a solvent control group-average tumor volume at the beginning of treatment in the solvent control group)] × 100%. The results were shown in Figure 13.

**Statistical Analysis:** Statistical analysis was performed using SPSS software based on relative tumor volume and tumor weight at the end of the experiment. Comparisons among multiple groups were analyzed by one-way ANOVA. If the variances were homogeneous (no significant difference in F-values), the analysis was performed by the Tukey's method, and if the variances were not homogeneous (significant difference in F-values), the test was performed by the Games-Howell method. P < 0.05 was considered as a significant difference.

**Experimental Results:** When administered to human lung cancer LU-01-0414 subcutaneous xenograft tumor for 14 days, the compound of Formula (I) showed significant inhibitory effects on tumor growth at three concentrations of 3, 15 and 30 mg/kg BID, with T/C of 9.57%, 3.07% and 1.87%, and TGI of 118.02%, 126.88% and 128.36%, respectively. P < 0.0001 compared to the solvent control group. When Crizotinib was at the 30 and 50 mg/kg QD dose groups, T/C = 10.32%, 4.89%, and TGI = 117.67%, 124.09%. P <0.0001 compared to the solvent control. Crizotinib also showed significant inhibitory effects on tumors. The above results suggest that in a human LU-01-0414 lung cancer nude mouse xenograft tumor model, the compound of Formula (I) had a significant anti-tumor effect at a dosage as low as 3 mg/kg, and the anti-tumor effect of the compound of Formula (I) at a dosage of 3 mg/kg was comparable to that of Crizotinib at a dosage of 30 mg/kg (p > 0.05).

## Claims

1. Crystal form A of a compound of Formula (I), 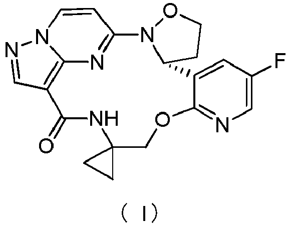 **characterized in that** an X-ray powder diffraction pattern of the crystal form A has characteristic diffraction peaks at the 2θ angels of 10.12°±0.20°, 19.03°±0.20°, and 19.74°±0.20°.

2. The crystal form A according to claim 1, wherein the X-ray powder diffraction pattern of the crystal form A has characteristic diffraction peaks at the 2θ angels of 10.12±0.20°, 17.74±0.20°, 18.18±0.20°, 19.03±0.20°, 19.74±0.20°, 20.75±0.20°, 24.60±0.20°, and 28.21±0.20°.

3. The crystal form A according to claim 2, wherein the X-ray powder diffraction pattern of the crystal form A has characteristic diffraction peaks at the 2θ angels of 10.115°, 17.740°, 18.178°, 19.033°, 19.738°, 20.749°, 21.609°, 22.411°, 23.345°, 24.160°, 24.597°, 25.787°, 28.211°, 30.613°, 31.133°, and 36.782°.

4. The crystal form A according to claim 3, wherein the XRPD pattern of the crystal form A is shown as Figure 1.

5. The crystal form A according to any one of claims 1-4, wherein a differential scanning calorimetry curve of the crystal form A has an onset of an endothermic peak at 171.4±3.0°C, and an onset of another endothermic peak at 221.1±3.0°C; and has a peak of an exothermic peak at 179.9±3.0°C.

6. The crystal form A according to claim 5, wherein a DSC pattern of the crystal form A is shown as Figure 2.

7. The crystal form A according to any one of claims 1-4, wherein a thermogravimetric analysis curve of the crystal form A has a weight loss of 2.26% at 200.0°C±3.0°C.

8. The crystal form A according to claim 7, wherein a TGA pattern of the crystal form A is shown as Figure 3.

9. Crystal form B of a compound of Formula (I), 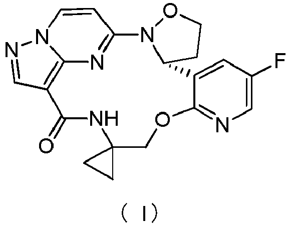 **characterized in that** an X-ray powder diffraction pattern of the crystal form B has characteristic diffraction peaks at the 2θ angels of 10.10±0.20°, 20.42±0.20°, and 22.21±0.20°.

10. The crystal form B according to claim 9, wherein the X-ray powder diffraction pattern of the crystal form B has characteristic diffraction peaks at the 2θ angels of 10.10±0.20°, 14.04±0.20°, 17.48±0.20°, 18.20±0.20°, 20.42±0.20°, 22.21±0.20°, 23.83±0.20°, and 27.49±0.20°.

11. The crystal form B according to claim 10, wherein the X-ray powder diffraction pattern of the crystal form B has characteristic diffraction peaks at the 2θ angels of 6.694°, 8.689°, 10.103°, 11.085°, 13.418°, 14.041°, 16.607°, 17.483°, 17.676°, 18.200°, 18.861°, 19.671°, 20.419°, 20.865°, 22.207°, 23.422°, 23.833°, 24.327°, 25.017°, 25.421°, 26.310°, 26.947°, 27.489°, 28.202°, 28.544°, 29.351°, 30.204°, 30.573°, 31.245°, 31.617°, 32.244°, 33.505°, 34.046°, 36.059°, 37.310°, and 38.534°.

12. The crystal form B according to claim 11, wherein the XRPD pattern of the crystal form B is shown as Figure 4.

13. The crystal form B according to any one of claims 9-12, wherein a differential scanning calorimetry curve of the crystal form B has an onset of an endothermic peak at 220.9±3.0°C.

14. The crystal form B according to claim 13, wherein a DSC pattern of the crystal form B is shown as Figure 5.

15. The crystal form B according to any one of claims 9-12, wherein a thermogravimetric analysis curve of the crystal form B has a weight loss of 1.16% at 200.0°C±3.0°C.

16. The crystal form B according to claim 15, wherein a TGA pattern of the crystal form B is shown as Figure 6.

17. Crystal form C of a compound of Formula (I), 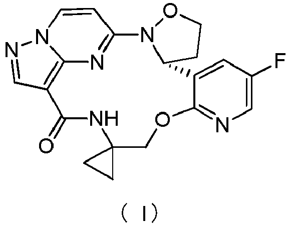 **characterized in that** an X-ray powder diffraction pattern of the crystal form C has characteristic diffraction peaks at the 2θ angels of 8.89°±0.20°, 13.37°±0.20°, and 20.98°±0.20°.

18. The crystal form C according to claim 17, wherein the X-ray powder diffraction pattern of the crystal form C has characteristic diffraction peaks at the 2θ angels of 8.89±0.20°, 12.86±0.20°, 13.37±0.20°, 14.71±0.20°, 18.58±0.20°, 20.98±0.20°, 21.85±0.20°, and 26.85±0.20°.

19. The crystal form C according to claim 18, wherein the X-ray powder diffraction pattern of the crystal form C has characteristic diffraction peaks at the 2θ angels of 8.889°, 12.862°, 13.373°, 14.706°, 17.376°, 17.950°, 18.584°, 20.146°, 20.640°, 20.977°, 21.497°, 21.847°, 22.912°, 25.959°, and 26.853°.

20. The crystal form C according to claim 19, wherein the XRPD pattern of the crystal form C is shown as Figure 7.

21. The crystal form C according to any one of claims 17-20, wherein a differential scanning calorimetry curve of the crystal form C has an onset of an endothermic peak at 215.8±3.0°C.

22. The crystal form C according to claim 21, wherein a DSC pattern of the crystal form C is shown as Figure 8.

23. The crystal form C according to any one of claims 17-20, wherein a thermogravimetric analysis curve of the crystal form C has a weight loss of 0.94% at 200.0°C±3.0°C.

24. The crystal form C according to claim 23, wherein a TGA pattern of the crystal form C is shown as Figure 9.

25. A method for preparing the crystal form C of the compound of Formula (I), comprising a gas-solid permeation method, a suspension stirring method and a slow cooling method.

26. The method according to claim 25, wherein the gas-solid permeation method comprises:
1) placing the compound of Formula (I) in a glass vial; and
2) placing the glass vial in an atmosphere of a solvent and then sealing the glass vial, allowing the solvent and the solid to induce crystallization,
wherein the solvent is methanol, ethyl acetate or acetone.

27. The method according to claim 25, wherein the suspension stirring method comprises:
1) formulating the compound of Formula (I) into a suspension in a solvent; and
2) slurrying the suspension at room temperature or 50°C,
wherein the solvent is methanol/water (v/v, 1:3), 1,4-dioxane/n-heptane (v/v, 1:4), dichloromethane/methyl tert-butyl ether (v/v, 1:9), anisole, isopropanol, isopropanol/water (v/v, 98:2), isopropanol/water (v/v, 94:6), isopropanol/water (v/v, 85:15), isopropyl acetate, ethanol/water (v/v, 1:3), methyl isobutyl ketone/n-heptane (v/v, 1:4), ethyl acetate/n-pentanol (v/v, 1:3), acetonitrile/methyl tert-butyl ether (v/v, 1:5), tetrahydrofuran/pure water (v/v, 1:9), chloroform/n-heptane (v/v, 1:5), or methyl ethyl ketone/methyl tert-butyl ether (v/v, 1:2); and the slurrying time is 2 hours to 144 hours.

28. The method according to claim 25, wherein the slow cooling method comprises:
1) formulating the compound of Formula (I) into a suspension in a solvent at 50°C;
2) stirring the suspension for 2 hours, filtering the suspension, and collecting a clear filtrate; and
3) cooling the clear filtrate from 50 °C to 5 °C at 0.1 °C/min;
wherein the solvent is isopropyl acetate.

29. Use of the crystal form A according to any one of claims 1-8, or the crystal form B according to any one of claims 9-16, or the crystal form C according to any one of claims 17-24, or the crystal forms obtained by the methods according to any one of claims 25-28 in the preparation of a medicament for treating solid tumors.
